# EUROPEAN PATENT SPECIFICATION

(11) **EP 4 079 311 B1**
(45) Date of publication and mention of the grant of the patent: **01.10.2025**
(21) Application number: 21169363.5
(22) Date of filing: 20.04.2021
(51) Int. Cl.: A61K 31/706, A61P 25/16, A61K 31/7084, A61P 25/24

(54) **NMN AND DERIVATIVES FOR USE IN THE TREATMENT OF DEPRESSION AND/OR ANXIETY IN PATIENTS HAVING A FORM OF PARKINSONISM**
NMN UND DERIVATE ZUR VERWENDUNG BEI DER BEHANDLUNG VON DEPRESSION UND/ODER ANGST BEI PATIENTEN MIT EINER FORM VON PARKINSONISMUS
NMN ET DÉRIVÉS POUR LEUR UTILISATION DANS LE TRAITEMENT DE LA DÉPRESSION ET/OU DE L'ANXIÉTÉ CHEZ DES PATIENTS AYANT UNE FORME DE LA MALADIE DE PARKINSON

(43) Date of publication of application: 26.10.2022
(73) Proprietor: Nuvamid SA, 1003 Lausanne (CH)
(72) Inventor: BERMOND, Guillaume, 13007 MARSEILLE (FR); GARCON, Laurent, 13960 SAUSSET LES PINS (FR)
(74) Representative: August Debouzy

(56) References cited:
- EP-A1- 3 323 415
- WO-A1-2016/149277
- WO-A1-2017/147058
- WO-A1-2019/152416
- WO-A1-2020/113811
- WO-A2-2006/105440
- KISS TAMAS ET AL: "Nicotinamide mononucleotide (NMN) treatment attenuates oxidative stress and rescues angiogenic capacity in aged cerebromicrovascular endothelial cells: a potential mechanism for the prevention of vascular cognitive impairment", GEROSCIENCE, SPRINGER INTERNATIONAL PUBLISHING, CHAM, vol. 41, no. 5, 29 May 2019 (2019-05-29), pages 619 - 630, XP036952852, ISSN: 2509-2715, [retrieved on 20190529], DOI: 10.1007/S11357-019-00074-2
- LEI LU ET AL: "Nicotinamide mononucleotide improves energy activity and survival rate in an in vitro model of Parkinson's disease", EXPERIMENTAL AND THERAPEUTIC MEDICINE, 14 July 2014 (2014-07-14), GR, XP055311369, ISSN: 1792-0981, DOI: 10.3892/etm.2014.1842
- CHANDRASEKARAN KRISH ET AL: "Nicotinamide Mononucleotide Administration Prevents Experimental Diabetes-Induced Cognitive Impairment and Loss of Hippocampal Neurons.", INTERNATIONAL JOURNAL OF MOLECULAR SCIENCES 26 MAY 2020, vol. 21, no. 11, 26 May 2020 (2020-05-26), XP009529952, ISSN: 1422-0067
- SHENGJUN WANG ET AL: "Design, Synthesis and SAR Studies of NAD Analogues as Potent Inhibitors towards CD38 NADase", MOLECULES, vol. 19, no. 10, 29 October 2014 (2014-10-29), DE, pages 15754 - 15767, XP055707855, ISSN: 1433-1373, DOI: 10.3390/molecules191015754
- SCHWARZ J ET AL: "Depression in Parkinson's disease", JOURNAL OF NEUROLOGY, STEINKOPFF-VERLAG, DE, vol. 258, no. 2, 11 May 2011 (2011-05-11), pages 336 - 338, XP019901208, ISSN: 1432-1459, DOI: 10.1007/S00415-011-6048-3
- LU ET AL.: "Nicotinamide mononucleotide improves energy activity andsurvival rate in an in vitro model of Parkinson's diseas", EXPERIMENTAL AND THERAPEUTIC MEDICINE 8: 943-950, 2014, 2014
- XIE ET AL.: "Nicotinamide mononucleotide ameliorates the depression-like behaviorsand is associated with attenuating the disruption of mitochondrialbioenergetics in depressed mice", JOURNAL OF AFFECTIVE DISORDERS 263 (2020) 166-174, 2020

## Description

### FIELD OF THE INVENTION

The invention relates to compounds of formula (I) and/or (la) according to the list of compounds claimed and at least one pharmaceutically acceptable excipient for use in the prevention and/or treatment of depression related to parkinsonism chosen amongst Parkinson disease (PD), dementia with Lewy bodies (DLB), progressive supranuclear palsy (PSP), cortico-basal degeneration (CBD), drug induced parkinsonism (DIP), vascular parkinsonism (VP) and multiple system atrophy (MSA).

### TECHNICAL BACKGROUND

Parkinsonism refers to a group of neurological disorders characterised by tremors, bradykinesia or slow movement, impaired speech, muscle stiffness and rigidity, postural instability. Within this group of disorders, idiopathic parkinsonism, or Parkinson's Disease is the most commonly identified in about 85% of patients. Atypical parkinsonism disorders such as dementia with Lewy bodies (DLB), progressive supranuclear palsy (PSP), cortico-basal degeneration (CBD), drug induced parkinsonism (DIP), vascular parkinsonism (VP) and multiple system atrophy (MSA) are more difficult to treat. Consequently, the psychological impact of these diseases, such as depression and/or anxiety, on patients is important and requires proper treatment.

Among parkinsonism, alpha-synucleinopathies are a group of neurodegenerative diseases characterised by the abnormal accumulation of aggregates of alpha-synuclein protein in neurons, nerve fibres or glial cells. The alpha-synucleinopathies comprise Parkinson's disease (PD), dementia with Lewy bodies (DLB) and multisystemic atrophy (MSA).

Parkinson's disease (PD) is a long-term degenerative disorder of the central nervous system that mainly affects the motor system. The symptoms usually emerge slowly and, as the disease worsens, non-motor symptoms become more common. The most obvious early symptoms are tremor, rigidity, slowness of movement, and difficulty with walking. Cognitive and behavioural problems may also occur with depression, anxiety, and apathy occurring in many people with PD. PD dementia becomes common in the advanced stages of the disease. PD patients can also experience problems with their sleep and sensory systems. Parkinson's disease (PD) is classified as G20 in the 10^{th} International Classification of Diseases (ICD 10) as published in the Official Bulletin No 2020/9 bis(https://www.atih.sante.fr/sites/default/files/public/content/3706/cim-10 2020 bo.pdf).

Dementia with Lewy bodies (DLB) is classified as G31.8 in the 10^{th} International Classification of Diseases (ICD 10). This disease is characterized by changes in thinking, movement, behaviour, and mood. Dementia with Lewy bodies and Parkinson's disease dementia have similar neuropathological features, but these features are highly variable, and the conditions cannot be distinguished on pathological features alone. Generally, dementia with Lewy bodies is distinguished from Parkinson's disease dementia by the time frame in which dementia symptoms appear relative to parkinsonian symptoms and is diagnosed when cognitive symptoms begin before or at the same time as parkinsonism. Parkinson's disease dementia is the diagnosis when Parkinson's disease is already well established before the dementia occurs. Between 5% and 25% of diagnosed dementias in older adults are due to Lewy body dementias. DLB usually develops after the age of 50. Men are more likely to be diagnosed than women. There is currently no treatment for these patients. Administration of L-dopamine does not alleviate significantly their symptoms.

Multisystemic Atrophy (MSA) is a rapidly progressing neurodegenerative disease with a sporadic adult prevalence of between 3.4 and 4.9 per 100,000 people. MSA is classified as atypical parkinsonism with symptoms including bradykinesia, rigidity and postural instability (postural hypotension). Other non-motor symptoms include urogenital, gastrointestinal and cardiovascular insufficiency. Sleep, cognitive impairment, respiratory problems and behavioural symptoms also occur during the progression of the disease. MSA is classified in the category G23 in ICD 10. MSA is defined in two subtypes, the Parkinsonian subtype (G23.2 in ICD 10) with patients with Parkinsonian-type symptoms, and the cerebellar subtype (G23.3 in ICD 10) for which patients suffer from cerebellar ataxia. MSA is characterized in part by abnormal accumulation of alpha-synuclein in oligodendrocytes, inflammation, demyelination and synaptic and neuronal loss. Infiltration of immune cells into brain tissue leads to significant inflammation. alpha-extracellular synuclein plays an essential role in the activation of microglia, which leads to the secretion of inflammatory cytokines and thus to neurodegeneration.

Patients suffering from idiopathic and atypical parkinsonism such as Parkinson's disease (PD), DLB, PSP, CBD, DIP, VP or MSA also suffers from depression due to the lack of effective treatments, especially for MSA, PSP, CBD, DIP, VP and DLB patients, as well as the gradual loss of autonomy and speech as the disease progress.

Because some symptoms of atypical parkinsonism are similar to those of Parkinson's disease (idiopathic parkinsonism), treatment with levodopa (L-dopamine), with or without carbidopa, which is an effective treatment of Parkinson disease (PD), has been tried for patients diagnosed with MSA, DLB PSP, CBD, DIP or VP. However, one of the downsides of Levodopa (L-dopamine) is that although the patients' mood is improved immediately after L-dopamine administration, it deteriorates along the day as L-dopamine is eliminated by the body. Document SCHWARZ J ET AL: "Depression in Parkinson's disease",JOURNAL OF NEUROLOGY, STEINKOPFF-VERLAG, DE, vol. 258, no. 2, 11 May 2011 (2011-05-11), pages 336-338, XP019901208,ISSN: 1432-1459, DOI: 10.1007/S00415-011-6048-3
the only well powered clinical trial addressing depressive symptoms in patients with Parkinson disease showed that the dopamine agonist pramipexole is highly effective to treat such symptom.

There is therefore a need to develop new treatments for depression related to parkinsonism such as Parkinson disease (PD), dementia with Lewy bodies (DLB), progressive supranuclear palsy (PSP), cortico-basal degeneration (CBD), drug induced parkinsonism (DIP), vascular parkinsonism (VP) and multiple system atrophy (MSA). There is also a need to develop such treatments which are well tolerated by patients, do not worsen any of their other symptoms, and compatible with a chronic administration.

### SUMMARY OF THE INVENTION

These goals are achieved, at least in part, by the present invention. Only the subject matter of the claims is part of the present invention.

When methods of treatment of the human or animal body are referred to, what is meant are compounds and compositions for use in such methods of treatment.

The present invention relates to specific compounds as in the list of the claims, which generally fall under formula (I): 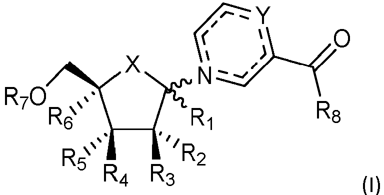 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 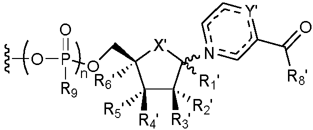 wherein n is an integer chosen amongst 1, 2 or 3; wherein:
   R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
   R11 is selected from C1-C10 alkyl, C3-C10 cycloalkyl, C5-C18 aryl, C1-C10 alkylaryl, substituted C5-C12 aryl, C1-C10 heteroalkyl, C3_C10 heterocycloalkyle, C1-C10 haloalkyl, heteroaryl, -(CH2)nC(O)(C1-C15)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of R11a is independently selected from H and (C1-C6) alkyl and R11b is independently selected from OH, C1-C6 alkoxy, NH2, NH(C1-C6 alkyl) or N(C1-C6 alkyl)2 ;
   R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
   R13 and R14 are independently selected from H, C1-C8 alkyl and C1-C8 alkyl aryl;
   Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
   R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
   R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
   R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl ;
   Y is selected from CH, CH2, C(CH3)2 and CCH3;
   -̅ -̅ -̅ represents a single or double bond according to Y; and
   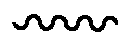 represents the alpha or beta anomer depending on the position of R1,
   or a compound of formula (la) 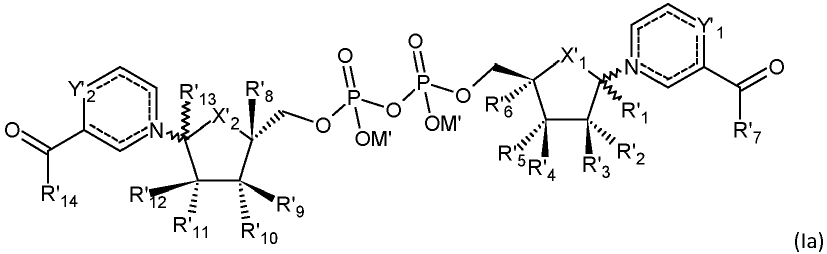
   or pharmaceutically acceptable salts, hydrates, crystals, stereoisomers and/or solvates thereof, wherein:
      X'1 and X'2 are independently selected from O, CH2, S, Se, CHF, CF2 and C=CH2;
      R'1 and R'13 are independently selected from H, azido, cyano, C1-C8 alkyl, C1-C8 thio-alkyl, C1-C8 heteroalkyl and OR; wherein R is selected from H and C1-C8 alkyl;
      R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid;
      R'6 and R'8 are independently selected from H, azido, cyano, C1-C8 alkyl and OR; wherein R is selected from H and C1-C8 alkyl;
      R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
      Y'1 and Y'2 are independently selected from CH, CH2, C(CH3)2 or CCH3;
      M' is selected from H or a suitable counter-ion;
      -̅ -̅ -̅ represents a single or a double bound depending on Y'1 and Y'2; and
      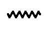 represents the alpha or beta anomer depending on the position of R'1 and R'13,
      and their combinations for their use in the prevention and/or treatment of depression and/or anxiety related to parkinsonism.

Advantageously, the compound according to the invention is selected from compounds I-A to I-J from Table 1 below ci-dessous or a pharmaceutically acceptable salt or solvate thereof:

**[Table 1]**

| **Composés (anomères)** | **Structure** |
|---|---|
| I-A (beta) | 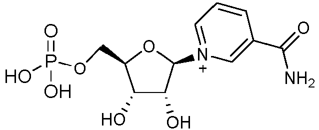 |
| I-B (alpha) | 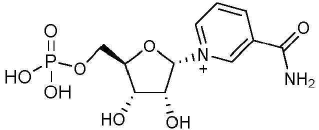 |
| I-C (beta) | 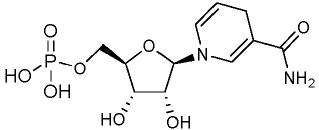 |
| I-D (alpha) | 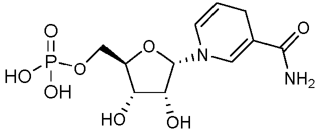 |
| I-E (beta) | 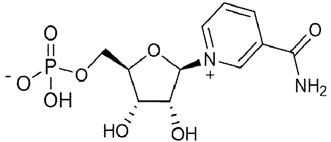 |
| I-F (alpha) | 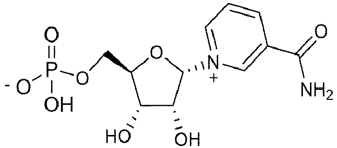 |
| I-G (beta) | 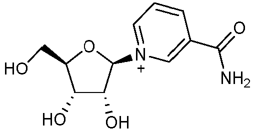 |
| I-H (alpha) | 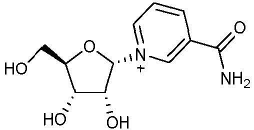 |
| I-I (beta) | 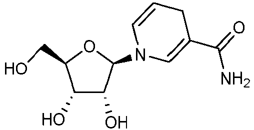 |
| I-J (alpha) | 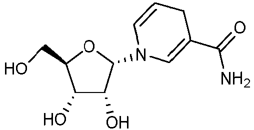 |

Advantageously, preferred compound of the invention are compounds I-A to I-F or a pharmaceutically acceptable salt or solvate thereof, more preferably IB, ID or IF.

Advantageously, the compound of formula (I) is dihydro-nicotinamide mononucleotide (NMN-H) of the following formula:

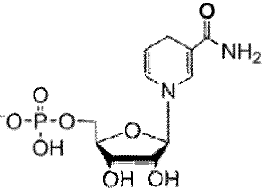

Advantageously, compounds of the invention are compounds la-A to Ia-I, listed in table 2:

**[Table 2]**

| **Compound** | **Structure** |
|---|---|
| Ia-A (beta, beta) | 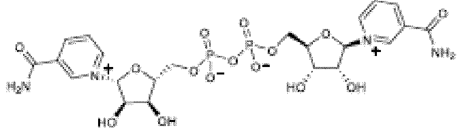 |
| Ia-B (beta, alpha) | 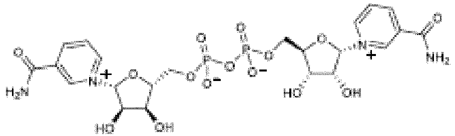 |
| Ia-C (alpha, alpha) | 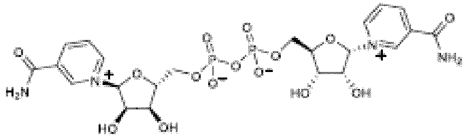 |
| Ia-D (beta, beta) | 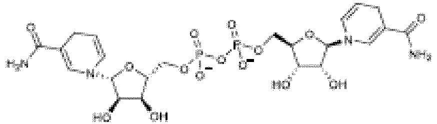 |
| Ia-E (beta, alpha) | 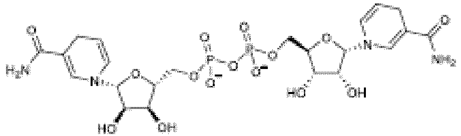 |
| Ia-F (alpha, alpha) | 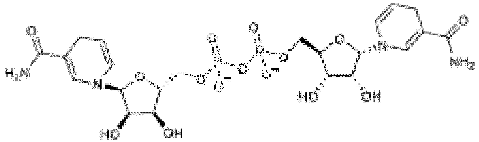 |
| Ia-G (beta, beta) | 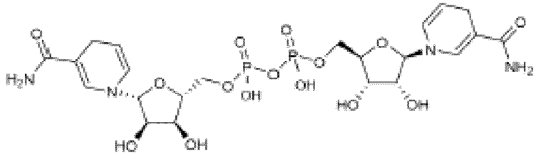 |
| Ia-H (beta, alpha) | 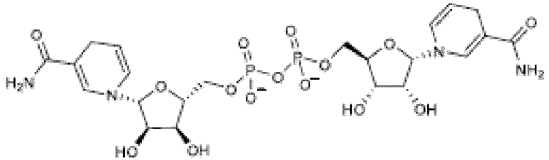 |
| Ia-I (alpha, alpha) | 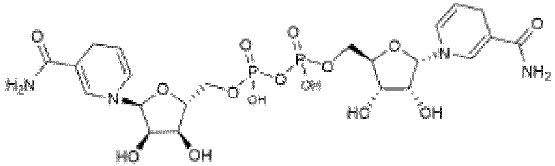 |

Advantageously, preferred compound of the invention is compound of formula la-C or la-F or Ia-I.

Advantageously, preferred compound of the invention is compound of formula la-B or la-E or la-H.

Advantageously, the parkinsonism can be chosen amongst an alpha-synucleinopathy, a progressive supranuclear palsy, a cortico-basal degeneration, a drug induced parkinsonism and a vascular parkinsonism

Advantageously, the alpha-synucleinopathy is chosen amongst Parkinson's disease, MSA and DLB.

In an embodiment, the alpha-synucleinopathy is MSA.

Preferably, the MSA is chosen amongst MSA-predominant Parkinsonism (MSA-P) and/or the MSA-predominant cerebellar ataxia (MSA-C).

In an embodiment, the alpha-synucleinopathy is Parkinson disease.

Advantageoulsy, the symptoms of depression related to parkinsonism can be chosen amongst anxiety, depression, sadness, anger, the sensation of lack of support from the loved ones, the feeling of being ignored and their combinations.

Advantageously, compound of formula (I) as described herein and/or compounds of formula (la) as described herein are for use in the treatment of depression related to parkinsonism as disclosed herein.

Advantageously, compound of formula I and/or compound of formula la may be used in an amount between 0.01 mg/kg/day and 1000 mg/kg/day, preferably between 1 mg/kg/day and 100 mg/kg/day, more preferably between 5 mg/kg/day and 50 mg/kg/day, even more preferably between 10 and 20 mg/kg/day.

Advantageously, compound of formula I and/or compound of formula la may be administered orally, ocularly, sublingually, parenterally, transcutaneously, vaginally, epidurally, intravesically, rectally or by inhalation.

Preferably, compound of formula I and/or compound of formula la may be administered orally or parenterally.

Advantageously, compound of formula I and/or compound of formula la may be used in mammals, preferably humans.

Advantageously, compound of formula I and/or compound of formula la may be used in combination with at least one further therapeutic ingredient.

Advantageously, the at least one further therapeutic ingredient may be chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations. Preferably, the further therapeutic ingredient is levodopa (L-dopamine), with or without carbidopa.

Preferably, the further therapeutic ingredient is levodopa (L-dopamine).

The present invention also relates to a composition comprising at least one compound of formula I and/or compound of formula la as described herein, one of its pharmaceutically acceptable salts and at least one pharmaceutically acceptable excipient for use in the prevention and/or treatment of depression related to parkinsonism.

Advantageously, the parkinsonism can be chosen amongst an alpha-synucleinopathy, a progressive supranuclear palsy, a cortico-basal degeneration, a drug induced parkinsonism and a vascular parkinsonism.

Advantageously, the parkinsonism is an alpha-synucleinopathy.

In an embodiment, the alpha-synucleinopathy is preferably MSA.

Preferably, the MSA is chosen amongst MSA-predominant Parkinsonism (MSA-P) and/or the MSA-predominant cerebellar ataxia (MSA-C).

In an embodiment, the alpha-synucleinopathy is Parkinson disease (PD).

Advantageously, the composition according to the invention is in the form of a tablet, a capsule, a sachet, a granule, a soft capsule, a lozenge, a lyophilisate, a suspension, a gel, a syrup, a solution, a water/oil emulsion, an oil/water emulsion, oil, cream, milk, spray, ointment, ampoule, suppository, eye drops, vaginal egg, vaginal capsule, liquid for inhalation, dry powder inhaler, pressurised metered dose inhaler.

Preferably, the composition according to the invention is in the form of a gastro-resistant capsule or a sublingual tablet.

Advantageously, the composition according to the invention is a pharmaceutical composition.

Advantageously, the composition according to the invention is a dietary supplement.

The present invention also relates to a composition comprising compound of formula I and/or compound of formula la, one of their pharmaceutically acceptable salts, at least one pharmaceutically acceptable excipient and at least one further therapeutic ingredient for its use according to the invention.

Advantageously, the at least one further therapeutic ingredient is as described herein.

The present invention also relates to a kit of parts comprising at least compound of formula I and/or compound of formula la, one of their pharmaceutically acceptable salts, and at least one further part.

Advantageously, the at least one further part may be an information leaflet.

Advantageously, the at least one further part may be at least one further therapeutic ingredient as described herein.

Preferably, the at least one further part comprises an information leaflet and at least one further therapeutic ingredient as described herein.

Advantageously, the kit of parts can be used in the treatment and/or prevention of depression and/or anxiety in parkinsonism.

Advantageoulsy, the parkinsonism is chosen amongst an alpha-synucleinopathy, a progressive supranuclear palsy, a cortico-basal degeneration, a drug induced parkinsonism and a vascular parkinsonism.

Advantageoulsy, the alpha-synucleinopathy is chosen amongst Parkinson's disease (PD), dementia with Lewy bodies (DLB) and multiple system atrophy (MSA) as described herein. In an embodiment, the alpha-synucleinopathy is multiple system atrophy (MSA). In one embodiment, the alpha-synucleinopathy is Parkinson's disease (PD).

Preferably, the kit of parts is used in the treatment and/or prevention of an alpha-synucleinopathy chosen amongst dementia with Lewy bodies (DLB) and multiple system atrophy (MSA) as described herein.

### DEFINITIONS

The definitions and explanations below are for the terms as used throughout the entire application, including both the specification and the claims.

When describing the compounds of the invention, the terms used are to be construed in accordance with the following definitions, unless indicated otherwise.

Unless indicated otherwise, the nomenclature of substituents that are not explicitly defined herein are arrived at by naming the adjacent functionality toward the point of attachment followed by the terminal portion of the functionality. For example, the substituent "arylalkyl" refers to the group -(aryl)-(alkyl).

In the present invention, the following terms have the following meanings.

The term "alkyl" by itself or as part of another substituent refers to a hydrocarbyl radical of Formula CnH2n+1 wherein n is a number greater than or equal to 1. Generally, alkyl groups of this invention comprise from 1 to 12 carbon atoms, preferably from 1 to 8 carbon atoms, more preferably from 1 to 6 carbon atoms, still more preferably 1 to 2 carbon atoms. Alkyl groups may be linear or branched and may be substituted as indicated herein. Suitable alkyl groups include methyl, ethyl, n-propyl, i-propyl, n- butyl, i-butyl, s-butyl and t-butyl, pentyl and its isomers (e.g. n-pentyl, iso-pentyl), and hexyl and its isomers (e.g. n-hexyl, iso-hexyl). Preferred alkyl groups include methyl, ethyl, n-propyl, i-propyl, n-butyl, i-butyl, s-butyl, t-butyl, n-pentyl, n-hexyl, n-heptyl, n-octyl, n-nonyl and n-decyl. Saturated branched alkyls include, without being limited to, isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl.

Suitable alkyl groups include methyl, ethyl, n-propyl, i-propyl, n-butyl, i-butyl, s-butyl and t-butyl, pentyl and its isomers (e.g. n-pentyl, iso-pentyl), hexyl and its isomers (e.g. n-hexyl, isohexyl), heptyl and its isomers (e.g. heptyl-heptyl, iso-heptyl), octyl and its isomers (e.g. n-octyl, iso-octyl), nonyl and its isomers (e.g. n-nonyl, iso-nonyl), decyl and its isomers (e.g. n-decyl, iso-decyl), undecyl and its isomers, dodecyl and its isomers. Preferred alkyl groups are methyl, ethyl, n-propyl, i-propyl, n-butyl, i-butyl, s-butyl and t-butyl. Cx-Cy-alkyl refers to alkyl groups which comprise x to y carbon atoms, x and y being included.

When the suffix "ene" ("alkylene") is used in conjunction with an alkyl group, this is intended to mean the alkyl group as defined herein having two single bonds as points of attachment to other groups. The term "alkylene" includes methylene, ethylene, methylmethylene, propylene, ethylethylene, and 1,2-dimethylethylene.

The term "alkenyl" as used herein refers to an unsaturated hydrocarbyl group, which may be linear or branched, comprising one or more carbon-carbon double bonds. Suitable alkenyl groups comprise between 2 and 12 carbon atoms, preferably between 2 and 8 carbon atoms, still more preferably between 2 and 6 carbon atoms. Examples of alkenyl groups are ethenyl, 2- propenyl, 2-butenyl, 3-butenyl, 2-pentenyl and its isomers, 2-hexenyl and its isomers, 2,4-pentadienyl and the like.

The term "alkynyl" as used herein refers to a class of monovalent unsaturated hydrocarbyl groups, wherein the unsaturation arises from the presence of one or more carbon-carbon triple bonds. Alkynyl groups typically, and preferably, have the same number of carbon atoms as described above in relation to alkenyl groups. Non limiting examples of alkynyl groups are ethynyl, 2- propynyl, 2-butynyl, 3-butynyl, 2-pentynyl and its isomers, 2-hexynyl and its isomers-and the like.

The term "aryl" as used herein refers to a polyunsaturated, aromatic hydrocarbyl group having a single ring (i.e. phenyl) or multiple aromatic rings fused together (e.g. naphtyl) or linked covalently, typically containing 5 to 12 atoms; preferably 6 to 10, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Non- limiting examples of aryl comprise phenyl, biphenylyl, biphenylenyl, 5- or 6-tetralinyl, naphthalen-1- or -2-yl, 4-, 5-, 6 or 7-indenyl, 1- 2-, 3-, 4- or 5- acenaphtylenyl, 3-, 4- or 5-acenaphtenyl, 1- or 2-pentalenyl, 4- or 5-indanyl, 5-, 6- , 7- or 8-tetrahydronaphthyl, 1,2,3,4-tetrahydronaphthyl, 1,4-dihydronaphthyl, 1-, 2-, 3-, 4- or 5-pyrenyl.

The term "cycloalkyl" as used herein is a cyclic alkyl group, that is to say, a monovalent, saturated, or unsaturated hydrocarbyl group having 1 or 2 cyclic structures. Cycloalkyl includes monocyclic or bicyclic hydrocarbyl groups. Cycloalkyl groups may comprise 3 or more carbon atoms in the ring and generally, according to this invention comprise from 3 to 10, more preferably from 3 to 8 carbon atoms still more preferably from 3 to 6 carbon atoms. Examples of cycloalkyl groups include but are not limited to cyclopropyl, cyclobutyl, cyclopentyl, cyclohexyl, with cyclopropyl being particularly preferred.

As used herein, the term "antibody" means a polypeptide comprising a framework region from an immunoglobulin gene or fragments thereof that specifically binds and recognizes an antigen, e.g., myostatin. Use of the term antibody is meant to include whole antibodies, including single-chain whole antibodies, and antigen-binding fragments thereof. The term "antibody" includes bispecific antibodies and multispecific antibodies so long as they exhibit the desired biological activity or function.

As used herein, the term "antibody-related polypeptide" means antigen-binding antibody fragments, including single-chain antibodies, that can comprise the variable region(s) alone, or in combination, with all or part of the following polypeptide elements: hinge region, CH1, CH2, and CH3 domains of an antibody molecule. Also included in the disclosure are any combinations of variable region(s) and hinge region, CH1, CH2, and CH3 domains. Antibody- molecules of the present technology include, e.g., but are not limited to, Fab, Fab' and F(ab')2, Fd, single-chain Fvs (scFv), single-chain antibodies, disulfide-linked Fvs (sdFv) and fragments comprising either a V L or V H domain. Examples include: (i) a Fab fragment, a monovalent fragment consisting of the VL, VH, CL and CH1 domains; (ii) a F(ab')z fragment, a bivalent fragment comprising two Fab fragments linked by a disulphide bridge at the hinge region; (iii) a Fd fragment consisting of the VH and CH1 domains; (iv) a Fv fragment consisting of the VL and VH domains of a single arm of an antibody, (v) a dAb fragment (Ward, et al., Nature 341: 544-546, 1989), which consists of a V H domain; and (vi) an isolated complementarity determining region (CDR). As such "antibody fragments" can comprise a portion of a full-length antibody, generally the antigen binding or variable region thereof. Examples of antibody fragments include Fab, Fab', F(ab')2, and Fv fragments; diabodies; linear antibodies; single-chain antibody molecules; and multispecific antibodies formed from antibody fragments. Single-chain antibody molecules may comprise a polymer with a number of individual molecules, for example, dimmer, trimer or other polymers.

As used herein, the term "CDR-grafted antibody" means an antibody in which at least one CDR of an "acceptor" antibody is replaced by a CDR "graft" from a "donor" antibody possessing a desirable antigen specificity.

As used herein, the term "chimeric antibody" means an antibody in which the Fc constant region of a monoclonal antibody from one species (e.g., a mouse Fc constant region) is replaced, using recombinant DNA techniques, with an Fc constant region from an antibody of another species (e.g., a human Fc constant region). See generally Better, et al., Science 240: 1041-1043, 1988; Liu, et al., Proc Natl Acad Sci USA 84: 3439-3443, 1987; Liu, et al., J Immuno/139: 3521-3526, 1987; Sun, et al., Proc Natl Acad Sci USA 84: 214-218, 1987; Nishimura, et al., Cancer Res 47:999-1005, 1987; Wood, et al., Nature 314:446-449, 1985; and Shaw, et al., JNatl Cancer Inst 80: 1553-1559, 1988.

As used herein, the term "diabodies" refers to small antibody fragments with two antigen-binding sites, which fragments comprise a heavy-chain variable domain (VH) connected to a light-chain variable domain (VL) in the same polypeptide chain (VH VL). By using a linker that is too short to allow pairing between the two domains on the same chain, the domains are forced to pair with the complementary domains of another chain and create two antigen binding sites. Diabodies are described more fully in Hollinger, et al., Proc. Natl. Acad. Sci. USA, 90: 6444-6448 (1993).

As used herein, the term "gene" means a segment of DNA that contains all the information for the regulated biosynthesis of an RNA product, including promoters, exons, introns, and other untranslated regions that control expression.

As used herein, the term "human sequence antibody" includes antibodies having variable and constant regions (if present) derived from human germline immunoglobulin sequences. The human sequence antibodies of the present technology can include amino acid residues not encoded by human germline immunoglobulin sequences (e.g., mutations introduced by random or site-specific mutagenesis in vitro or by somatic mutation in vivo). Such antibodies can be generated in non-human transgenic animals, e.g., as described in PCT Publication Nos. WO 01/14424 and WO 00/37504. However, the term "human sequence antibody," as used herein, is not intended to include antibodies in which CDR sequences derived from the germline of another mammalian species, such as a mouse, have been grafted onto human framework sequences (e.g., humanized antibodies).

As used herein, the term "humanized" forms of non-human (e.g., murine) antibodies are chimeric antibodies which contain minimal sequence derived from non-human immunoglobulin. For the most part, humanized antibodies are human immunoglobulins in which hypervariable region residues of the recipient are replaced by hypervariable region residues from a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance such as binding affinity. Generally, the humanized antibody comprises substantially all of at least one, and typically two, variable domains, in which all or substantially all of the hypervariable loops correspond to those of a non-human immunoglobulin and all or substantially all of the FR regions are those of a human immunoglobulin sequence although the FR regions may include one or more amino acid substitutions that improve binding affinity. The number of these amino acid substitutions in the FR are typically no more than 6 in the H chain, and in the L chain, no more than 3. The humanized antibody optionally also comprises at least a portion of an immunoglobulin constant region (Fe), typically that of a human immunoglobulin. For further details, see Jones, et al., Nature 321:522-525 (1986); Reichmann, et al., Nature 332:323-329 (1988); and Presta, Curr. Op. Struct. Bioi. 2:593- 596 (1992).

As used herein, the term "hypervariable region" refers to the amino acid residues of an antibody which are responsible for antigen-binding. The hypervariable region generally comprises amino acid residues from a "complementarity determining region" or "CDR" (e.g. around about residues 24-34 (LI), 50-56 (L2) and 89-97 (L3) in the VL, and around about 3I- 35B (HI), 50-65 (H2) and 95-I02 (H3) in the VH (Kabat, et al., Sequences of Proteins of Immunological Interest, 5th Ed. Public Health Service, National Institutes of Health, Bethesda, MD. (1991)) and/or those residues from a "hypervariable loop" (e.g. residues 26-32 (LI), 50-52 (L2) and 91-96 (L3) in the VL, and 26-32 (HI), 52A-55 (H2) and 96-IOI (H3) in the VH (Chothia and Lesk J. Mol. Bioi. 196:901-917 (1987)).

As used herein, "gene therapy" refers to the therapeutic delivery of nucleic acids into a patient's cells as a drug to treat disease. The delivery of DNA into cells can be accomplished by multiple methods. The two major classes are recombinant viruses (sometimes called biological nanoparticles or viral vectors) and naked DNA or DNA complexes (non-viral methods).

The term "halo" or "halogen" means fluoro, chloro, bromo, or iodo. Preferred halo groups are fluoro and chloro.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoro methyl, 1,1,1-trifluoroethyl and the like. Cx-Cy-haloalkyl and Cx-Cy-alkyl are alkyl groups which comprise x to y carbon atoms. Preferred haloalkyl groups are difluoromethyl and trifluoromethyl.

Where at least one carbon atom in an aryl group is replaced with a heteroatom, the resultant ring is referred to herein as a heteroaryl ring.

The term "heteroalkyl" means an alkyl group as defined above in which one or more carbon atoms are replaced by a heteroatom selected from oxygen, nitrogen and sulfur atoms. In heteroalkyl groups, the heteroatoms are linked along the alkyl chain only to carbon atoms, i.e. each heteroatom is separated from any other heteroatom by at least one carbon atom. However, the nitrogen and sulphur heteroatoms may optionally be oxidised and the nitrogen heteroatoms may optionally be quaternised. A heteroalkyl is bonded to another group or molecule only through a carbon atom, i.e. the bonding atom is not selected from the heteroatoms included in the heteroalkyl group.

The term "heteroaryl" as used herein by itself or as part of another group refers but is not limited to 5 to 12 carbon-atom aromatic rings or ring systems containing 1 to 2 rings which are fused together or linked covalently, typically containing 5 to 6 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2, 1 -b] [ 1,3] thiazolyl, thieno [3 ,2-b] furanyl, thieno [3 ,2-b] thiophenyl, thieno[2,3-d][I,3]thiazolyl, thieno[2,3-d]imidazolyl, tetrazolo[I,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2- benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3- benzoxadiazolyl, 1 ,2,3-benzothiadiazolyl, 2, 1 ,3-benzothiadiazolyl, thienopyridinyl, purinyl, imidazo[I,2-a]pyridinyl, 6-oxo-pyridazin-I(6H)-yl, 2- oxopyridin-I(2H)-yl, 6-oxo-pyridazin-I(6H)-yl, 2-oxopyridin-I(2H)-yl, 1,3- benzodioxolyl, quinolinyl, isoquinolinyl, cinnolinyl, quinazolinyl, quinoxalinyl.

Where at least one carbon atom in a cycloalkyl group is replaced with a heteroatom, the resultant ring is referred to herein as "heterocycloalkyl" or "heterocyclyl".

The terms "heterocyclyl", "heterocycloalkyl" or "heterocyclo" as used herein by itself or as part of another group refer to non-aromatic, fully saturated or partially unsaturated cyclic groups (for example, 3 to 7 member monocyclic, 7 to 11 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen, oxygen and/or sulfur atoms, where the nitrogen and sulfur heteroatoms may optionally be oxidized and the nitrogen heteroatoms may optionally be quaternized. Any of the carbon atoms of the heterocyclic group may be substituted by oxo (for example piperidone, pyrrolidinone). The heterocyclic group may be attached at any heteroatom or carbon atom of the ring or ring system, where valence allows. The rings of multi- ring heterocycles may be fused, bridged and/or joined through one or more spiro atoms. Non limiting exemplary heterocyclic groups include oxetanyl, piperidinyl, azetidinyl, 2-imidazolinyl, pyrazolidinyl imidazolidinyl, isoxazolinyl, oxazolidinyl, isoxazolidinyl, thiazolidinyl, isothiazolidinyl, piperidinyl, 3H-indolyl, indolinyl, isoindolinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, 3-dioxolanyl, 1,4-dioxanyl, 2,5-dioximidazolidinyl, 2- oxopiperidinyl, 2-oxopyrrolodinyl, indolinyl, tetrahydropyranyl, tetrahydrofuranyl, tetrahydroquinolinyl, tetrahydroisoquinolin- 1-yl, tetrahydroisoquinolin-2-yl, tetrahydroisoquinolin-3-yl, tetrahydroisoquinolin-4-yl, thiomorpholin-4-yl, thiomorpholin-4-ylsulf oxide, thiomorpholin-4-ylsulfone, 1,3- dioxolanyl, 1,4-oxathianyl, IH-pyrrolizinyl, tetrahydro-I,I-dioxothiophenyl, N- formylpiperazinyl, and morpholin-4-yl.

The term "non-proteinogenic amino acid" as used herein refers to an amino acid not naturally encoded or found in the genetic code of living organism. Non limiting examples of Non-proteinogenic amino acid are ornithine, citrulline, argininosuccinate, homoserine, homocysteine, cysteine-sulfinic acid, 2-aminomuconic acid, δ-aminolevulinic acid, β-alanine, cystathionine, γ-aminobutyrate, DOPA, 5-hydroxytryptophan, D-serine, ibotenic acid, α-aminobutyrate, 2-aminoisobutyrate, D-leucine, D-valine, D-alanine or D-glutamate .

The term "proteinogenic amino acid" as used herein refers to an amino acid that is incorporated into proteins during translation of messenger RNA by ribosomes in living organisms, i.e. Alanine (ALA), Arginine (ARG), Asparagine (ASN), Aspartate (ASP), Cysteine (CYS), Glutamate (glutamic acid) (GLU), Glutamine (GLN), Glycine (GLY), Histidine (HIS), Isoleucine (ILE), Leucine (LEU), Lysine (LYS), Methionine (MET), Phenylalanine (PHE), Proline (PRO), Pyrrolysine (PYL), Selenocysteine (SEL), Serine (SER), Threonine (THR), Tryptophan (TRP), Tyrosine (TYR) or Valine (VAL).

The term "prodrug" as used herein means the pharmacologically acceptable derivatives of compounds of formula (I) such as esters whose in vivo biotransformation product is the active drug. Prodrugs are characterized by increased bioavailability and are readily metabolized into the active compounds in vivo. Suitable prodrugs for the purpose of the invention include carboxylic esters, in particular alkyl esters, aryl esters, acyloxyalkyl esters, and dioxolene carboxylic esters; ascorbic acid esters.

The term "substituent" or "substituted" means that a hydrogen radical on a compound or group is replaced by any desired group which is substantially stable under the reaction conditions in an unprotected form or when protected by a protecting group. Examples of preferred substituents include, without being limited to, halogen (chloro, iodo, bromo, or fluoro); alkyl; alkenyl; alkynyl, as described above; hydroxy; alkoxy; nitro; thiol; thioether; imine; cyano; amido; phosphonato; phosphine; carboxyl; thiocarbonyl; sulfonyl; sulfonamide; ketone; aldehyde; ester; oxygen (-O); haloalkyl (e.g., trifluoromethyl); cycloalkyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic (e.g., pyrrolidinyl, piperidinyl, piperazinyl, morpholinyl, or thiazinyl), monocyclic or fused or non-fused polycyclic aryl or heteroaryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); CO2CH3; CONH2; OCH2CONH2; NH2; SO2NH2; OCHF2; CF3; OCF3; and such moieties may also be optionally substituted by a fused-ring structure or bridge, for example -OCH2O-. These substituents may optionally be further substituted with a substituent selected from such groups. In certain embodiments, the term "substituent" or the adjective "substituted" refers to a substituent selected from the group consisting of an alkyl, an alkenyl, an alkynyl, an cycloalkyl, an cycloalkenyl, a heterocycloalkyl, an aryl, a heteroaryl, an arylalkyl, a heteroarylalkyl, a haloalkyl, -C(O)NR11R12, -NR13C(O)R14, a halo, -OR13, cyano, nitro, a haloalkoxy, -C(O)R13, -NR11R12, -SR13, -C(O)OR13, -OC(O)R13, -NR13C(O)NR11R12, -OC(O)NR11R12, -NR13C(O)OR14, -S(O)rR13, -NR13S(O)rR14, -OS(O)rR14, S(O)rNR11R12, -O, -S, and -N-R13, wherein r is 1 or 2; R11 and R12, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl; or R11 and R12 taken together with the nitrogen to which they are attached is optionally substituted heterocycloalkyl or optionally substituted heteroaryl; and R13 and R14 for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl. In certain embodiments, the term "substituent" or the adjective "substituted" refers to a solubilizing group.

The term "active ingredient" or "therapeutic ingredient" refers to a molecule or a substance whose administration to a subject slows down or stops the progression, aggravation, or deterioration of one or more symptoms of a disease, or condition; alleviates the symptoms of a disease or condition; cures a disease or condition. According to one embodiment, the therapeutic ingredient is a small molecule, either natural or synthetic. According to another the therapeutic ingredient is a biological molecule such as for example an oligonucleotide, a siRNA, a miRNA, a DNA fragment, an aptamer, an antibody and the like.

By "pharmaceutically acceptable" is meant that the ingredients of a pharmaceutical composition are compatible with each other and not deleterious to the patient thereof.

The term "pharmaceutically acceptable excipient" or "pharmaceutical vehicle" refers to an inert medium or carrier used as a solvent or diluent in which the pharmaceutically active agent is formulated and/or administered, and which does not produce an adverse, allergic or other reaction when administered to an animal, preferably a human being. This includes all solvents, dispersion media, coatings, antibacterial and antifungal agents, isotonic agents, absorption retardants and other similar ingredients. For human administration, preparations must meet standards of sterility, general safety and purity as required by regulatory agencies such as the FDA or EMA. For the purposes of the invention, "pharmaceutically acceptable excipient" includes all pharmaceutically acceptable excipients as well as all pharmaceutically acceptable carriers, diluents, and/or adjuvants.

The term "pharmaceutically acceptable salts" include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts.

Hemisalts of acids and bases may also be formed, for example, hemisulphate and hemicalcium salts.

Pharmaceutically acceptable salts of compounds of Formula (I) may be prepared by one or more of these methods:
(i) by reacting the compound of Formula (I) with the desired acid;
(ii) by reacting the compound of Formula (I) with the desired base;
(iii) by removing an acid- or base-labile protecting group from a suitable precursor of the compound of Formula (I) or by ring-opening a suitable cyclic precursor, e.g., a lactone or lactam, using the desired acid; and/or
(iv) by converting one salt of the compound of Formula (I) to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost non-ionized.

Although generally, with respect to the salts of the compounds of the invention, pharmaceutically acceptable salts are preferred, it should be noted that the invention in its broadest sense also included non-pharmaceutically acceptable salts, which may for example be used in the isolation and/or purification of the compounds of the invention. For example, salts formed with optically active acids or bases may be used to form diastereoisomeric salts that can facilitate the separation of optically active isomers of the compounds of Formula I above.

The term "solvate" is used herein to describe a molecular complex comprising a compound of the invention and contains stoichiometric or sub-stoichiometric amounts of one or more pharmaceutically acceptable solvent molecule, such as ethanol. The term 'hydrate' refers to when said solvent is water.

The term "administration", or a variant thereof (e.g., "administering"), means providing the active agent or active ingredient, alone or as part of a pharmaceutically acceptable composition, to the patient in whom/which the condition, symptom, or disease is to be treated or prevented.

The term "human" refers to a subject of both genders and at any stage of development (i.e., neonate, infant, juvenile, adolescent, adult).

The term "patient" refers to a warm-blooded animal, more preferably a human, who/which is awaiting the receipt of, or is receiving medical care or is/will be the object of a medical procedure.

The terms "treat", "treating" and "treatment", as used herein, are meant to include alleviating, attenuating or abrogating a condition or disease and/or its attendant symptoms.

The terms "prevent", "preventing" and "prevention", as used herein, refer to a method of delaying or precluding the onset of a condition or disease and/or its attendant symptoms, barring a patient from acquiring a condition or disease, or reducing a patient's risk of acquiring a condition or disease.

The term "therapeutically effective amount" (or more simply an "effective amount") as used herein means the amount of active agent or active ingredient that is sufficient to achieve the desired therapeutic or prophylactic effect in the patient to which/whom it is administered.

The bonds of an asymmetric carbon can be represented here using a solid triangle ( 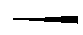 ), a dashed triangle ( 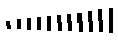 ) or a zigzag line ( 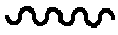 ).

### DETAILED DESCRIPTION OF THE INVENTION

The present invention thus relates to a compound of formula (I) : 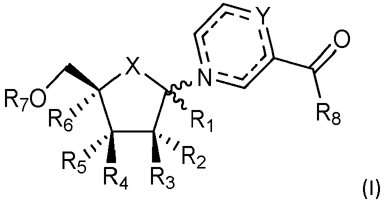 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 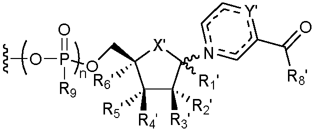 wherein n is an integer chosen amongst 1, 2 or 3; wherein:
   R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
   R11 is selected from C1-C10 alkyl, C3-C10 cycloalkyl, C5-C18 aryl, C1-C10 alkylaryl, substituted C5-C12 aryl, C1-C10 heteroalkyl, C3_C10 heterocycloalkyle, C1-C10 haloalkyl, heteroaryl, -(CH2)nC(O)(C1-C15)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of R11a is independently selected from H and (C1-C6) alkyl and R11b is independently selected from OH, C1-C6 alkoxy, NH2, NH(C1-C6 alkyl) or N(C1-C6 alkyl)2 ;
   R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
   R13 and R14 are independently selected from H, C1-C8 alkyl and C1-C8 alkyl aryl;
   Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
   R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
   R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
   R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl ;
   Y is selected from CH, CH2, C(CH3)2 and CCH3;
   -̅ -̅ -̅ represents a single or double bond according to Y; and
   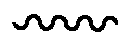 represents the alpha or beta anomer depending on the position of R1,
   or a compound of formula (la) 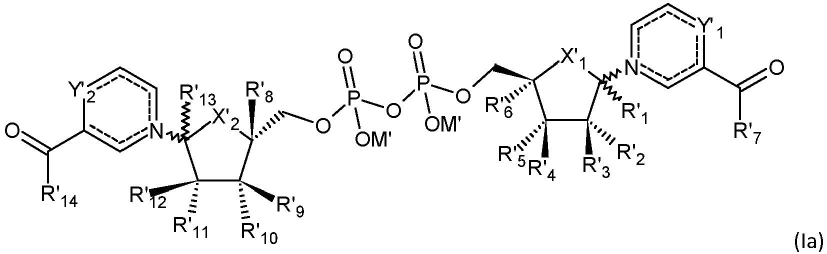
   or pharmaceutically acceptable salts, hydrates, crystals, stereoisomers and/or solvates thereof, wherein:
      X'1 and X'2 are independently selected from O, CH2, S, Se, CHF, CF2 and C=CH2;
      R'1 and R'13 are independently selected from H, azido, cyano, C1-C8 alkyl, C1-C8 thio-alkyl, C1-C8 heteroalkyl and OR; wherein R is selected from H and C1-C8 alkyl;
      R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid;
      R'6 and R'8 are independently selected from H, azido, cyano, C1-C8 alkyl and OR; wherein R is selected from H and C1-C8 alkyl;
      R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
      Y'1 and Y'2 are independently selected from CH, CH2, C(CH3)2 or CCH3;
      M' is selected from H or a suitable counter-ion; 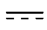
      represents a single or a double bound depending on Y'1 and Y'2; and 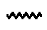
      represents the alpha or beta anomer depending on the position of R'1 and R'13,
      and their combinations for their use in the prevention and/or treatment of depression and/or anxiety in parkinsonism chosen amongst Parkinson's disease (PD), dementia with Lewy bodies (DLB), progressive supranuclear palsy (PSP), cortico-basal degeneration (CBD), drug-induces parkinsonism (DIP), vascular parkinsonism (VP) or multiple systemic atrophy (MSA).

Compounds of formula I and formula la are derivatives of nicotinamide mononucleotide. More specifically, compounds of formula I comprises NMN and its derivatives. Compounds of formula la are derivatives of nicotinamide mononucleotide (NMN).

Advantageously, X is selected from O, CH2 and S.

Advantageously, R1 is selected from hydrogen or OH. In one embodiment, R1 is hydrogen. In one embodiment, R1 is OH.

Advantageously, R2, R3, R4 and R5 are independently selected from hydrogen, halogen, hydroxyl, C1-C12 alkyl and OR; wherein R is as described herein above. In a preferred embodiment, R2, R3, R4 and R5 are independently selected from hydrogen, hydroxyl and OR; wherein R is as described herein above. In a more preferred embodiment R2, R3, R4 and R5 are independently selected from hydrogen or OH.

Advantageously, R2 and R3 are identical. In one embodiment, R2 and R3 are identical and represent OH. In one embodiment, R2 and R3 are identical and represent hydrogen.

Advantageously, R2 and R3 are different. In a preferred embodiment, R2 is hydrogen and R3 is OH. In a more preferred embodiment, R2 is OH and R3 is hydrogen.

Advantageously, R4 and R5 are identical. In one embodiment, R4 and R5 are identical and represent OH. In one embodiment, R4 and R5 are identical and represent hydrogen.

Advantageously, R2 and R3 are different. In a preferred embodiment, R4 is OH and R5 is hydrogen. In a more preferred embodiment, R4 is hydrogen and R5 is OH.

Advantageously, R3 and R4 are different. In one embodiment, R3 is OH and R4 is hydrogen. In one embodiment, R3 is hydrogen and R4 is OH.

Advantageously, R3 and R4 are identical. In a preferred embodiment, R3 and R4 are identical and represent OH. In a more preferred embodiment, R3 and R4 are identical and represent hydrogen.

Advantageously, R2 and R5 are different. In one embodiment, R2 is hydrogen and R5 is OH. In one embodiment, R2 is OH and R5 is hydrogen.

According to one embodiment, R2 and R5 are identical. In a preferred embodiment, R2 and R5 are identical and represent hydrogen. In a more preferred embodiment, R2 and R5 are identical and represent OH.

According to one embodiment, R6 is selected from hydrogen or OH. In one embodiment, R6 is OH. In a preferred embodiment, R6 is hydrogen.

Advantageously, R7 is selected from P(O)R9R10 or P(S)R9R10; wherein R9 and R10 are as described herein above. In a preferred embodiment, R7 is P(O)R9R10; wherein R9 and R10 are as described herein above. In a preferred embodiment, R7 is P(O)(OH)2.

Advantageously, R8 is selected from H, OR, NHR13 or NR13R14; wherein R13 and R14 are as described herein above. In a preferred embodiment, R8 is NHR13; wherein R13 and R14 are as described herein above.

Advantageously, Y is a CH or CH2. In one embodiment, Y is a CH. In one embodiment, Y is a CH2.

According to one preferred embodiment, compounds of formula (I) are those wherein X is an oxygen.

According to a preferred embodiment, the invention relates to compounds of general Formula (II): 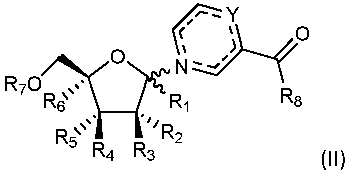
or a pharmaceutically acceptable salt or solvate thereof; wherein R1, R2, R3, R4, R5, R6, R7, R8, Y, -̅ -̅ -̅ and
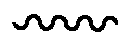 are as described herein above for compounds of formula (I).

Advantageously, preferred compounds of formula (I) are those wherein R1 is hydrogen.

Advantageously, preferred compounds of formula (I) are those wherein R2 is OH and R3 is hydrogen.

Advantageously, preferred compounds of formula (I) are those wherein R4 is hydrogen and R5 is OH.

Advantageously, preferred compounds of formula (I) are those wherein R3 and R4 are identical and represent hydrogen.

Advantageously, preferred compounds of formula (I) are those wherein R2 and R5 are identical and represent OH.

According to one embodiment, preferred compounds of formula (I) are those wherein R6 is hydrogen.

Advantageously, preferred compounds of formula (I) are those wherein R8 is NH2.

Advantageously, preferred compounds of formula (I) are those wherein Y is CH.

Advantageously, preferred compounds of formula (I) are those wherein Y is CH2.

Advantageously, preferred compounds of formula (I) are those wherein R7 is P(O)(OH)2.

Advantageously, the compound according to the invention is selected from compounds I-A to I-J from Table 1 below ci-dessous or a pharmaceutically acceptable salt or solvate thereof:

**[Table 1]**

| **Composés (anomères)** | **Structure** |
|---|---|
| I-A (beta) | 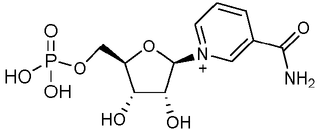 |
| I-B (alpha) | 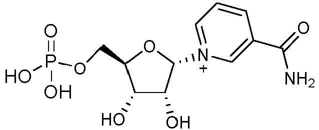 |
| I-C (beta) | 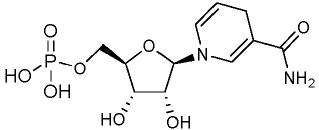 |
| I-D (alpha) | 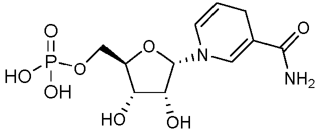 |
| I-E (beta) | 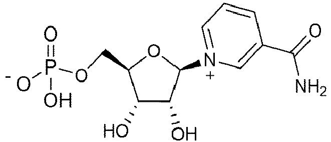 |
| I-F (alpha) | 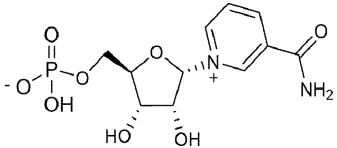 |
| I-G (beta) | 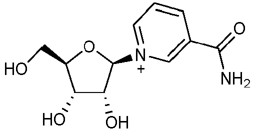 |
| I-H (alpha) | 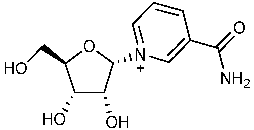 |
| I-I (beta) | 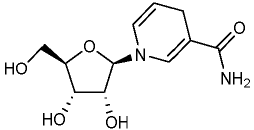 |
| I-J (alpha) | 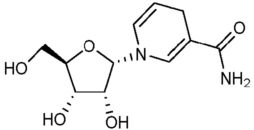 |

Advantageously, preferred compound of the invention are compounds I-A to I-F or a pharmaceutically acceptable salt or solvate thereof.

Advantageously, the pharmaceutically acceptable derivative is NMN-H of the following formula:

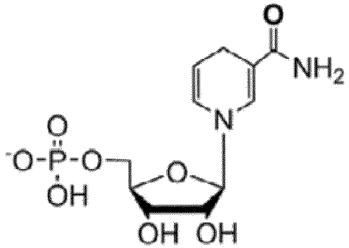

Advantageously, preferred compounds of general Formula la are those wherein X'1 and X'2 are independently selected from O, CH2, S.

Advantageously, R'7 and R'14 are independently selected from H, OR, NHR and NRR' wherein R and R' are independently selected from H, C1-C8 alkyl, C1-C8 alkyl aryl. According to one embodiment, R'7 and R'14 are NHR wherein R is selected from H, C1-C8 alkyl, C1-C8 alkyl aryl.

Advantageously, R'2, R'3, R'4, R'5, R'9, R'10, R'11, R'12 are independently selected from H, halogen, hydroxyl, C1-C12 alkyl and OR. According to a preferred embodiment, R'2, R'3, R'4, R'5, R'9, R'10, R'11, R'12 are independently selected from H, hydroxyl and OR, wherein R is as described herein above.

Advantageously, preferred compounds of general Formula la are those wherein, R'2, R'3, R'4, R'5, R'9, R'10, R'11, R'12 are independently selected from H and OH.

Advantageously, R'2 and R'3 are identical. According to one embodiment, R'2 and R'3 are identical and represent each a OH. According to one embodiment, R'2 and R'3 are identical and represent each hydrogen.

According to a preferred embodiment, R'2 and R'3 are different. According to a preferred embodiment, R'2 is hydrogen and R'3 is a OH. According to a more preferred embodiment, R'2 is a OH and R'3 is hydrogen.

Advantageously, R'4 and R'5 are identical. According to one embodiment, R'4 and R'5 are identical and represent each a OH. According to one embodiment, R'4 and R'5 are identical and represent each hydrogen.

According to a preferred embodiment, R'4 and R'5 are different. According to a preferred embodiment, R'4 is a OH and R'5 is hydrogen. According to a more preferred embodiment, R'4 is hydrogen and R'5 is a OH.

Advantageously, R'3 and R'4 are identical. According to one embodiment, R'3 and R'4 are identical and represent each a OH. According to one embodiment, R'3 and R'4 are identical and represent each hydrogen.

According to a preferred embodiment, R'3 and R'4 are different. According to a preferred embodiment, R'3 is a OH and R'4 is hydrogen. According to a more preferred embodiment, R'3 is hydrogen and R'4 is a OH

Advantageously, R'2 and R'5 are different. According to one embodiment, R'2 is hydrogen and R'5 is a OH. According to one embodiment, R'2 is a OH and R'5 is hydrogen.

According to a preferred embodiment, R'2 and R'5 are identical. According to a preferred embodiment, R'2 and R'5 are identical and represent each hydrogen. According to a more preferred embodiment, R'2 and R'5 are identical and represent each a OH.

Advantageously, R'9 and R'10 are identical. According to one embodiment, R'9 and R'10 are identical and represent each a OH. According to one embodiment, R'9 and R'10 are identical and represent each hydrogen.

According to a preferred embodiment, R'9 and R'10 are different. According to a preferred embodiment, R'9 is hydrogen and R'10 is a OH. According to a more preferred embodiment, R'9 is a OH and R'10 is hydrogen.

Advantageously, R'11 and R'12 are identical. According to one embodiment, R'11 and R'12 are identical and represent each a OH. According to one embodiment, R'11 and R'12 are identical and represent each hydrogen.

According to a preferred embodiment, R'11 and R'12 are different. According to a preferred embodiment, R'11 is a OH and R'12 is hydrogen. According to a more preferred embodiment, R'11 is hydrogen and R'12 is a OH.

Advantageously, R'10 and R'11 are different. According to one embodiment, R'10 is hydrogen and R'11 is a OH. According to one embodiment, R'10 is a OH and R'11 is hydrogen.

According to a preferred embodiment, R'10 and R'11 are identical. According to a preferred embodiment, R'10 and R'11 are identical and represent each a OH. According to a more preferred embodiment, R'10 and R'11 are identical and represent each hydrogen.

Advantageously, R'9 and R'12 are different. According to one embodiment, R'9 is hydrogen and R'12 is a OH. According to one embodiment, R'9 is a OH and R'12 is hydrogen.

According to a preferred embodiment, R'9 and R'12 are identical. According to a preferred embodiment, R'9 and R'12 are identical and represent each hydrogen. According to a more preferred embodiment, R'9 and R'12 are identical and represent each a OH.

Advantageously, Y'1 is CH. According to one embodiment, Y'1 is CH2.

Advantageously, Y'2 is CH. According to one embodiment, Y'2 is CH2.

Advantageously, X'1 and X'2 are different and are selected from the group as described above. According to one embodiment, X'1 and X'2 are identical and are selected from the group as described above.

Advantageously, preferred compounds of general Formula la are those wherein X'1 and X'2 each independently represents an Oxygen.

Advantageously, preferred compounds of general Formula la are those wherein X'1 and X'2 are identical and represent each an Oxygen.

Advantageously, R'7 and R'14 are different and are selected from the group as described above. According to one embodiment, R'7 and R'14 are identical and are selected from the group as described above.

Advantageously, preferred compounds of general Formula la are those wherein R'7 and R'14 each independently represents a NH2.

Advantageously, preferred compounds of general Formula la are those wherein R'7 and R'14 are identical and represent each a NH2.

Advantageously, R'1 and R'13 are different and are selected from the group as described above. According to one embodiment, R'1 and R'13 are identical and are selected from the group as described above.

Advantageously, preferred compounds of general Formula la are those wherein R'1 and R'13 each independently represents a hydrogen.

Advantageously, preferred compounds of general Formula la are those wherein R'1 and R'13 are identical and represent each a hydrogen.

Advantageously, R'6 and R'8 are different and are selected from the group as described above. According to one embodiment, R'6 and R'8 are identical and are selected from the group as described above.

Advantageously, preferred compounds of general Formula la are those wherein R'6 and R'8 each independently represents a hydrogen.

Advantageously, preferred compounds of general Formula la are those wherein R'6 and R'8 are identical and represent each a hydrogen.

Advantageously, R'3, R'4, R'10 and R'11 are different and are selected from the group as described above. According one embodiment, R'3, R'4, R'10 and R'11 are identical and are selected from the group as described above.

Advantageously, preferred compounds of general Formula la are those wherein R'3, R'4, R'10 and R'11 each independently represents a hydrogen.

Advantageously, preferred compounds of general Formula la are those wherein R'3, R'4, R'10, R'11 are identical and represent each a H.

Advantageously, R'2, R'5, R'9 and R'12 are different and are selected from the group as described above. According one embodiment, R'2, R'5, R'9 and R'12 are identical and are selected from the group as described above.

Advantageously, preferred compounds of general Formula la are those wherein R'2, R'5, R'9 and R'12 each independently represents a OH.

Advantageously, preferred compounds of general Formula la are those wherein R'2, R'5, R'9, R'12 are identical and represent each a OH.

Advantageously, Y'1 and Y'2 are different. According to a preferred embodiment, Y'1 and Y'2 are identical.

Advantageously, preferred compounds of general Formula la are those wherein Y'1 and Y'2 each independently represents a CH.

Advantageously, preferred compounds of general Formula la are those wherein Y'1 and Y'2 are identical and represent each a CH.

Advantageously, preferred compounds of general Formula la are those wherein Y'1 and Y'2 each independently represents a CH2.

Advantageously, preferred compounds of general Formula la are those wherein Y'1 and Y'2 are identical and represent each a CH2.

Advantageously, preferred compounds of the invention are compounds la-A to Ia-I, listed in table 2:

**[Table 2]**

| **Compound** | **Structure** |
|---|---|
| Ia-A (beta, beta) | 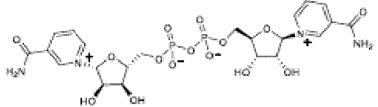 |
| Ia-B (beta, alpha) | 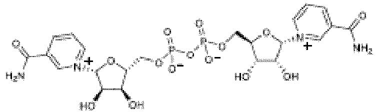 |
| Ia-C (alpha, alpha) | 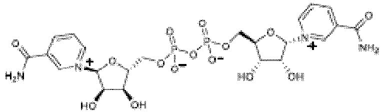 |
| Ia-D (beta, beta) | 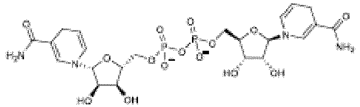 |
| Ia-E (beta, alpha) | 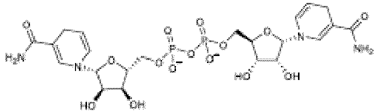 |
| Ia-F (alpha, alpha) | 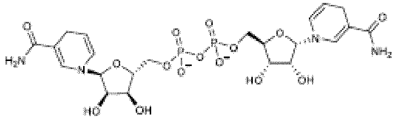 |
| Ia-G (beta, beta) | 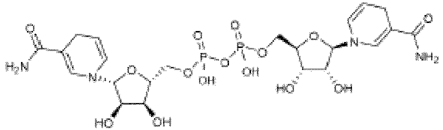 |
| Ia-H (beta, alpha) | 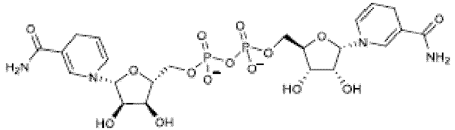 |
| Ia-I (alpha, alpha) | 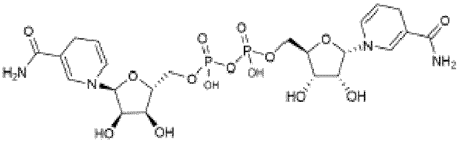 |

Advantageously, preferred compound of the invention is compound of formula la-C or la-F or Ia-I.

Advantageously, preferred compound of the invention is compound of formula la-B or la-E or la-H.

All references to compounds of Formula (I) or (la) include references to salts, solvates, multi component complexes and liquid crystals thereof. All references to compounds of Formula (I) or (la) include references to polymorphs and crystal habits thereof.

This invention also relates to the use of a compound of formula (I) and/or formula (la) as described herein in the treatment and/or prevention of depression and/or anxiety related to parkinsonism such as Parkinson's disease (PD), dementia with Lewy bodies (DLB), progressive supranuclear palsy (PSP), cortico-basal degeneration (CBD), drug-induces parkinsonism (DIP), vascular parkinsonism (VP) and multiple system atrophy (MSA).

### Therapeutic use

Compounds of formula I and/or of formula la as well as compositions and kit of parts comprising the same may be used in the treatment and/or prevention of depression and/or anxiety related to parkinsonism. Parkinsonism syndromes wherein the invention can be particularly useful are alpha-synucleinopathies such as Parkinson's disease (PD), dementia with Lewy bodies (DLB) and multiple system atrophy (MSA).

Indeed, the inventors have demonstrated that compounds of formula (I) and (la), unlike the conventional or experimental treatments of depression and/or anxiety related to parkinsonism so far tested, the use of the compounds of formula (I) and/or (la) according to the invention also provides good results on the signs of depression related to parkinsonism. Therefore, the invention provides an efficient treatment in order to reduce the symptoms of depression related to parkinsonism, such as anxiety, depression, sadness, anger, the sensation of lack of support from the loved ones, the feeling of being ignored and their combinations. In addition, the compounds of the invention are well tolerated and can be thus administered chronically, without involving any adverse events for the patients.

The autonomy and ability to carry out every day task can be assessed by the Unified Multiple System Atrophy Rating Scale (UMSARS) questionary and/or the MSA-QoL questionary. The UMSARS questionary has been developed by a group of physician (G.K.Wenning et al., Movement Disorders, Vol. 19, No. 12, 2004, pp. 1391-1402, DOI: 10.1002/mds.20255). The improvement of quality of life can be assessed by the use of the MSA-QoL questionary (Anette Schrag et al, Movement Disorders, 2007 Dec;22(16):2332-8; https://doi.org/10.1002/mds.21649). These questionnaires are often used for the evaluation of the quality of life of PD patients. However, they are also useful tools to evaluate the quality of life for patients having a form of parkinsonism.

The compounds of formula (I) or (la) as well as compositions and kit of part according to the invention are specifically useful to treat the depression related to parkinsonism such as Parkinson's disease (PD), dementia with Lewy bodies (DLB), progressive supranuclear palsy (PSP), cortico-basal degeneration (CBD), drug-induces parkinsonism (DIP), vascular parkinsonism (VP) and multiple system atrophy (MSA)., as demonstrated by the inventors.

Preferably, the parkinsonism is an alpha-synucleinopathy.

Preferably, the alpha-synucleinopathy is multiple system atrophy (MSA) or Parkinson's disease (PD).

In an embodiment, the compounds of formula (I) and/or compounds of formula (la) and compositions according to the invention are to be used in mammals, preferably humans. In an embodiment, the compounds of formula (I) or formula (la) and compositions according to the invention are to be used in male humans. In an embodiment, the compounds of formula (I) and/or compounds of formula (la) and compositions according to the invention are to be used in patients aged at least 18 or less than 18.

### Therapeutic combinations

Compounds of formula (I) and/or compounds of formula (la) as well as compositions comprising the same can be used in combinations with one or more further therapeutic ingredients conventionally used for the prevention and/or treatment of depression related to parkinsonism such as Parkinson's disease (PD), dementia with Lewy bodies (DLB), progressive supranuclear palsy (PSP), cortico-basal degeneration (CBD), drug induced parkinsonism (DIP), vascular parkinsonism (VP) and multiple system atrophy (MSA), preferably an alpha-synucleinopathy, preferably MSA or PD. More specifically, the invention can be used in combination with the conventional treatments of parkinsonism, such as PD or MSA.

Such at least one further therapeutic ingredient can be chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations. Preferably, the further therapeutic ingredient is levodopa (L-dopamine), with or without carbidopa.

The anticholinergic agent can be an antimuscarinic agent or an antinicotinic agent. The antimuscarinic agent can be chosen amongst clozapine, quietapine, atropine, benztropine, biperiden, chlorpherinamine, parotexine, dicyclomine, dimenhydrate, diphenhydramine, doxepin, doxylamine, glycopyrrolate, glycopyrronium, hyoscyamine, ipratropium, orphenadrine, oxitropium, oxybutynin, promethazine, propantheline bromide, scopolamine, solifenacin, tolterodine, tiotropium, tricyclic antidepressant, trihexyphenidyl, tropicamide, umeclidinium and their combinations, preferably diphenhydramine. The antinicotinic agent can be bupropion, dextromethorphan, doxacurium, hexamethonium, mecamylamine, tubocurarine and their combinations.

The acetylcholinesterase (ACE) inhibitor is chosen amongst benazepril, captopril, cilazapril, enalapril, fosinopril, imidapril, lisinopril, moexipril, perindopril, pyridostigmine, quinapril, ramipril, trandolapril, zofenopril and their combinations.

The tricyclic antidepressant can be amineptine, amitriptyline, amoxapine, butriptyline, clomipramine, desipramine, dibenzepin, dosulepin, doxepin, imipramine, iprindole, lofepramine, maprotiline, norclomipramine, northiaden, nortriptyline, opipramol, protriptyline, protriptyline, trimipramine, and their combinations. The benzodiazepine can be adinazolam, alprazolam, climazolam, clonazepam, clorazepate, diazepam, estazolam, flunitrazepam, flurazepam, halazepam, prazepam, loprazolam, lorazepam, lormetazepam, nimetazepam, nitrazepam, midazolam, oxazepam, temazepam, triazolam, and their combinations.

The dopaminergic agent can be a dopamine agonist, a dopamine antagonist, a dopamine transporter (DAT) inhibitor, a vesicular monoamine transporter (VMAT) inhibitor, a releasing agent, an enzyme inhibitor, and their combination.

The dopamine agonist can be chosen amongst:
- adamantanes: amantadine, memantine, rimantadine; and/or
- aminotetralins: 7-OH-DPAT, 8-OH-PBZI, rotigotine, UH-232; and/or
- benzazepines: 6-BR-APB, fenoldopam, SKF-38,393, SKF-77,434, SKF-81,297, SKF-82,958, SKF-83,959; and/or
- ergolines: bromocriptine, cabergoline, dihydroergocryptine, lisuride, lysergic acid diethylamide (Isd), pergolide; and/or
- dihydrexidine derivatives: 2-OH-NPA, A-86,929, ciladopa, dihydrexidine, dinapsoline, dinoxyline, doxanthrine: and/or
- A-68,930, A-77,636, A-412,997, ABT-670, ABT-724, aplindore, apomorphine, aripiprazole, bifeprunox, BP-897, CY-208,243, dizocilpine, etilevodopa, flibanserin, ketamine, melevodopa, modafinil, pardoprunox, phencyclidine, PD-128,907, PD-168,077, PF-219,061, piribedil, pramipexole, propylnorapomorphine, pukateine, quinagolide, quinelorane, quinpirole, RDS-127, RO10-5824, ropinirole, rotigotine, roxindole, salvinorin A, SKF-89,145, sumanirole, terguride, umespirone, WAY-100,635.

The dopamine antagonist can be chosen amongst:
- typical antipsychotics such as acepromazine, azaperone, benperidol, bromperidol, clopenthixol, chlorpromazine, chlorprothixene, droperidol, flupentixol, fluphenazine, fluspirilene, haloperidol, loxapine, mesoridazine, methotrimeprazine, nemonapride, penfluridol, perazine, periciazine, perphenazine, pimozide, prochlorperazine, promazine, sulforidazine, sulpiride, sultopride, thioridazine, thiothixene, trifluoperazine, triflupromazine, trifluperidol, zuclopenthixol; and/or
- atypical antipsychotics such as amisulpride, asenapine, blonanserin, cariprazine, carpipramine, clocapramine, clozapine, gevotroline, iloperidone, lurasidone, melperone, molindone, mosapramine, ocaperidone, olanzapine, paliperidone, perospirone, piquindone, quetiapine, remoxipride, risperidone, sertindole, tiospirone, ziprasidone, zotepine; and/or
- antiemetics such as AS-8112, alizapride, bromopride, clebopride, domperidone, metoclopramide, thiethylperazine; and/or
- amoxapine, buspirone, butaclamol, ecopipam, n-ethoxycarbonyl-2-ethoxy-1,2-dihydroquinoline (EEDQ); eticlopride, fananserin, L-745,870, nafadotride, nuciferine, PNU-99,194, raclopride, sarizotan, SB-277,011-A, SCH-23,390, SKF-83,566, SKF-83,959, sonepiprazole, spiperone, spiroxatrine, stepholidine, tetrahydropalmatine, tiapride, UH-232, yohimbine.

The dopamine transporter (DAT) inhibitor can be chosen amongst:
- piperazine such as DBL-583, GBR-12,935, nefazodone, vanoxerine, and/or
- piperidine such as 1-(1-(1-Benzothiophen-2-yl)cyclohexyl)piperidine (BTCP), desoxypipradrol, dextromethylphenidate, difemetorex, ethylphenidate, methylnaphthidate, isopropylphenidae, methylphenidate, phencyclidine, pipradrol; and/or
- pyrrolidine such as diphenylprolinol, methylenedioxypyrovalerone (mdpv), naphyrone, prolintane, pyrovalerone; and/or
- tropanes such as β-(4'-Chlorophenyl)-2β-(3'-phenylisoxazol-5'-yl)tropane (β-CPPIT), altropane, brasofensine , WIN 35428 (β-CFT) , cocaine, dichloropane , difluoropine , N-(2'-Fluoroethyl-)-3β-(4'-chlorophenyl)-2β-(3'-phenylisoxazol-5'-yl)nortropane (FE-β-CPPIT) , N-(3'-Fluoropropyl-)-3β-(4'-chlorophenyl)-2β-(3'-phenylisoxazol-5'-yl)nortropane (FP-β-CPPIT) , loflupane (123l) , lometopane , RTI-112 , RTI-113 , RTI-121 , RTI-126 , RTI-150 , RTI-177 , RTI-229 , RTI-336 , tenocyclidine , tesofensine, troparil , tropoxane, 2β-Propanoyl-3β-(4-tolyl)-tropane (WF-11), 2β-Propanoyl-3β-(2-naphthyl)-tropane (WF-23) , 2-Propanoyl-3-(4-isopropylphenyl)-tropane (WF-31) , 2α-(Propanoyl)-3β-(2-(6-methoxynaphthyl))-tropane (WF-33); and/or
- others such as adrafinil, armodafinil, amfonelic acid, amineptine, benzatropine (benztropine), Bromantane, 2-Butyl-3-(p-tolyl)quinuclidine (BTQ), BTS-74,398, bupropion (amfebutamone), ciclazindol, diclofensine, dimethocaine, diphenylpyraline, dizocilpine, DOV-102,677, DOV-21,947, DOV-216,303, etybenzatropine (ethylbenztropine), EXP-561, fencamine, fencamfamine, fezolamine, GYKI-52,895, hydrafinil, indatraline, ketamine, lefetamine, levophacetoperane, LR-5182, manifaxine, mazindol, medifoxamine, mesocarb, modafinil, nefopam, nomifensine, NS-2359, O-2172, pridefrine, propylamphetamine, radafaxine, SEP-225,289, SEP-227,162, sertraline, sibutramine, tametraline, tripelennamine.

The vesicular monoamine transporter (VMAT) inhibitor can be deserpidine, ibogaine, reserpine and/or tetrabenazine.

The releasing agent can be chosen amongst:
- morpholine such as fenbutrazate, morazone, phendimetrazine, phenmetrazine; and/or
- oxazoline such as 4-methylaminorex (4-mar, 4-max), aminorex, clominorex, cyclazodone, fenozolone, fluminorex, pemoline, thozalinone; and/or
- phenethylamines (also amphetamines, cathinones, phentermines, etc.) such as 2-hydroxyphenethylamine (2-OH-PEA), 4-chlorophenylisobutylamine (4-CAB), 4-methylamphetamine (4-MA), 4-methylmethamphetamine (4-MMA), alfetamine , amfecloral , amfepentorex, amfepramone, amphetamine (dextroamphetamine, levoamphetamine), amphetaminil, β-methylphenethylamine, benzodioxolylbutanamine (bdb), benzodioxolylhydroxybutanamine , benzphetamine, buphedrone, butylone, cathine, cathinone, clobenzorex, clortermine, d-deprenyl, dimethoxyamphetamine (dma), dimethoxymethamphetamine (DMMA), dimethylamphetamine, dimethylcathinone (dimethylpropion, metamfepramone), ethcathinone (ethylpropion), ethylamphetamine, ethylbenzodioxolylbutanamine (EBDB) , ethylone , famprofazone, fenethylline, fenproporex, flephedrone, fludorex, furfenorex, hordenine, lophophine (homomyristicylamine) , mefenorex, mephedrone, methamphetamine (desoxyephedrine, methedrine; dextromethamphetamine, levomethamphetamine), methcathinone (methylpropion), methedrone, methoxymethylenedioxyamphetamine (MMDA), methoxymethylenedioxymethamphetamine (mmdma), methylbenzodioxolylbutanamine (MBDB) , methylenedioxyamphetamine (mda, tenamfetamine), methylenedioxyethylamphetamine (MDEA), methylenedioxyhydroxyamphetamine, methylenedioxymethamphetamine, methylenedioxymethylphenethylamine, methylenedioxyphenethylamine, methylone, ortetamine, parabromoamphetamine, parachloroamphetamine (pca) , parafluoroamphetamine , parafluoromethamphetamine, parahydroxyamphetamine, paraiodoamphetamine, paredrine (norpholedrine, oxamphetamine), phenethylamine, pholedrine, phenpromethamine , prenylamine , propylamphetamine, tiflorex (flutiorex), tyramine, xylopropamine, zylofuramine

- piperazine such as 2,5-dimethoxy-4-bromobenzylpiperazine, benzylpiperazine, methoxyphenylpiperazine (meopp, paraperazine), methylbenzylpiperazine (MBZP), methylenedioxybenzylpiperazine (MDBZP, piperonylpiperazine); and/or
- others such as 2-amino-1,2-dihydronaphthalene, 2-aminoindane, 2-aminotetralin, 4-benzylpiperidine, 5-iodo-2-aminoindane, clofenciclan, cyclopentamine, cypenamine, cyprodenate, feprosidnine, gilutensin, heptaminol, hexacyclonate, indanylaminopropane, indanorex, isometheptene, methylhexanamine, naphthylaminopropane, octodrine, phthalimidopropiophenone, propylhexedrine (levopropylhexedrine), tuaminoheptane (tuamine).

The enzyme inhibitors can be a phenylalanine hydroxylase inhibitor such as 3,4-dihydroxystyrene, a tyrosine hydroxylase inhibitors such as 3-iodotyrosine, aquayamycin, bulbocapnine, metirosine and/or oudenone, an aromatic L-amino acid decarboxylase (DOPA) inhibitor such as benserazide, carbidopa, genistein, methyldopa, a monoamine oxidase (MAO) inhibitor, a catechol-O-methyl transferase (COMT) inhibitor such as entacapone, nitecapone, opicapone, tolcapone, a dopamine beta hydroxylase inhibitor such as bupicomide, disulfiram, dopastin, fusaric acid, nepicastat, phenopicolinic acid and tropolone, and their combinations.

Compounds and compositions according to the invention can be administered with the further therapeutic ingredient simultaneously, separately, or sequentially. By "simultaneously" is meant that two agents are administered concurrently. By "separately" is meant that the gap between the administration of the first agent and that of the second is substantial. By "sequentially" is meant that the two agents are administered one after the other within a time frame such that they are both available to act therapeutically within the same time frame. The optimum time interval between administering the two agents will vary depending on the precise nature of the method for delivering the compounds or compositions of the invention.

### Composition

The present invention also relates to a composition comprising compound of formula I and/or compound of formula la as described hereinabove and at least one pharmaceutically acceptable excipient for its use in the prevention and/or treatment of depression and/or anxiety related to a parkinsonism such as dementia with Lewy bodies (DLB), progressive supranuclear palsy (PSP), cortico-basal degeneration (CBD), drug-induced parkinsonism (DIP), vascular parkinsonism (VP) and multiple system atrophy (MSA), preferably MSA, as described herein.

In one embodiment, the composition according to the invention is a pharmaceutical composition. In another embodiment, the composition according to the invention is a dietary supplement. Preferably, the composition according to the invention is a pharmaceutical composition.

In one embodiment, the pharmaceutical composition according to the invention may be used in combination with at least one further therapeutic ingredient as described herein. In another embodiment, the pharmaceutical composition according to the invention comprises as least one further therapeutic ingredient as described herein.

The compositions according to the invention may be formulated with carriers, excipients and diluents which are suitable in themselves for these formulations, such as lactose, dextrose, saccharose, sorbitol, mannitol, starches, acacia gum, calcium phosphate, alginates, tragacanth gum, gelatine, calcium silicate, microcrystalline cellulose, polyvinylpyrrolidone, polyethylene glycol, cellulose, water (sterile), methyl cellulose, methyl and propyl hydroxybenzoates, talc, magnesium stearate, edible oils, vegetable and mineral oils or suitable mixtures thereof. The formulations may possibly contain other substances that are commonly used in pharmaceutical formulations, such as lubricants, wetting agents, emulsifying and suspending agents, dispersing agents, disintegrants, fillers, preservatives, sweeteners, flavourings, flow regulators, mould release agents, etc. The compositions can also be formulated to allow a rapid, prolonged or delayed release of the active compound(s) they contain.

The compositions according to the invention are preferably in the form of unit doses and may be packaged in an appropriate manner, for example in a box, blister pack, bottle, sachet, ampoule or in any other suitable single-dose or multiple-dose carrier or container (which may be properly labelled); optionally with one or more leaflet(s) containing product information and/or instructions for use.

Compositions of the invention can be prepared by any method known by the skilled in the art.

### Methods of administration

The compounds of formula (I) and formula (la) as well as compositions of the invention as described herein, may be administered by oral, parenteral (e.g., intramuscular, intraperitoneal, intravenous, intracerebroventricular, intracisternal injection or infusion, subcutaneous injection, or implant), by inhalation spray, nasal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals, such as mice, rats, horses, cattle, sheep, dogs, cats, monkeys, etc., the compounds of the invention are effective for use in humans. The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The compositions may be in a form suitable for oral use, for example, as tablets, troches, lozenges, aqueous or oily suspensions, dispersible powders or granules, emulsions, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material, such as glyceryl monostearate or glyceryl distearate may be employed. They may also be coated by the techniques described in the U.S. Patents 4,256,108; 4,166,452; and 4,265,874 to form osmotic therapeutic tablets for control release. Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy- propylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol , such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin. Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavoring agents may be added to provide a palatable oral preparation. These compositions may be preserved by the addition of an anti-oxidant, such as ascorbic acid. Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Syrups and elixirs may be formulated with sweetening agents, for example glycerol, propylene glycol, sorbitol or sucrose. Such formulations may also contain a demulcent, a preservative and flavouring and colouring agents.

The compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids, such as oleic acid find use in the preparation of injectables. The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols. For topical use, creams, ointments, jellies, solutions or suspensions, etc., containing the compounds of the present invention are employed. (For purposes of this application, topical application shall include mouthwashes and gargles.)

An appropriate dosage level is generally about 0.01 to 500 mg per kg patient body weight per day which can be administered in single or multiple doses. Preferably, the dosage level is about 0.1 to about 350 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. For example, the dosage may comprise from 100mg/day to 5000mg/day, preferably from 500mg/day to 1000mg/day.

"Gastro-resistant" means a galenic form that does not dissolve in the stomach. Such dosage forms are delayed release, i.e. they have a coating or coating composition that is resistant to the acid pH of the stomach (pH<2) to dissolve in the intestine. The gastroresistant character is determined by following the test established by the European Pharmacopoeia. Briefly, the gastroresistant character of a capsule is measured in 0.1 M hydrochloric acid at 37°C as a disintegrating medium in a disintegrating apparatus. This medium mimics the physicochemical condition of the stomach. The capsules are incubated in this medium for 1 hour. The capsule must show no signs of disintegration or cracks that could lead to a loss of contents. The capsule is then incubated for 1 hour in a phosphate buffer solution of pH 6.8 at 37°C, this solution mimics the conditions of the intestinal environment in accordance with the recommendations of the European Pharmacopoeia. The capsule must be completely disintegrated within one hour.

"Sublingual tablet" means a galenic form that is placed under the tongue so that the active principle is absorbed by the sublingual mucosa, and in particular by the ranin veins and arteries.

The galenic form of the composition according to the invention may also be immediate release: such a galenic form allows rapid absorption of NMN, its salts or derivative, and thus a reduced time of action. Immediate release galenic forms include dispersible, orodispersible, effervescent tablets and oral lyophilisates.

The galenic form of the composition according to the invention may also be delayed release. Dissolution and absorption of the compounds of formula (I) and/or formula (Ia) of the invention takes place in the intestine, which limits gastric irritation or degradation of the fragile active ingredients at acidic pH. These are mainly gastroresistant forms, i.e. the tablets or granules are covered with a polymeric film, insoluble in an acidic medium but permeable to water in an alkaline medium or of the lipidic type degraded by intestinal lipases.

The galenic form of the composition according to the invention may also be a prolonged and sequential release form. The forms with sequential release (release at precise time intervals) and prolonged release (continuous release of the active principle until exhaustion) promote the spreading of the release of the active principle over time in order to maintain an effective plasma concentration for a longer period of time in the patient's body. Such galenic forms make it possible to obtain relief of the patient's pain for a longer period of time and to space out the medication intake.

The mode of administration and the galenic form are determined by the person in the profession according to the anatomical location of the pain to be treated and the patient. Reference is made to the latest edition of Remington's Pharmaceutical Sciences.

Compounds of formula (I) and/or compounds of formula (la) as described herein and the composition according to the invention may be administered between 1 to 4 times per day, preferably once, twice or three times per day. Once or twice a day has been found suitable. Preferably, the compounds and compositions of the invention are to be taken before breakfast.

The duration of the treatment depends from the patent and is determined by the physician. It can be from one day to one year or even longer, preferably from one week to three months, more preferably from two weeks to six weeks. It will be understood, however, that the specific dose level and frequency of dosage and duration for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

### Kit of parts

The present invention also relates to a kit of parts as in the claims comprising at least compound of formula I and/or compound of formula la or a composition of the invention as described hereinabove, and at least one further part.

In one embodiment, the at least one further part may be an information leaflet which may comprise information to the user and/or instructions for use. In one embodiment, the at least one further part may be at least one further therapeutic ingredient as set out above. Preferably, the at least one further part comprises an information leaflet and at least one further therapeutic ingredient as described herein.

The kit of parts is used in the treatment and/or prevention of depression and/or anxiety related to parkinsonism such as dementia with Lewy bodies (DLB) and multiple system atrophy (MSA), preferably MSA as described hereinabove.

### Synthesis of compounds of the invention

According to another aspect, a method for the preparation of the compound of Formula (I) as described above is presently disclosed. Compounds of formula (I) can also be prepared according to the methods disclosed in international patent application WO 2017/079195 A1 and US patent US 10,611,790 B2.

In particular, the compounds of Formula (I) disclosed herein may be prepared as described below from substrates A-E.

The method can involve in a first step the mono-phosphorylation of a compound of formula (A), in the presence of phosphoryl chloride and a trialkyl phosphate, to yield the phosphorodichloridate of formula (B), 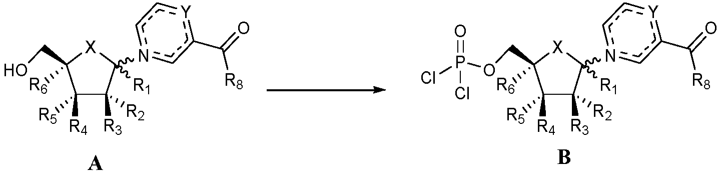 wherein X, R1, R2, R3, R4, R5, R6, R7, R8, Y, -̅ -̅ -̅ and 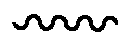 are as described herein above for compounds of formula (I).

In a second step, the phosphorodichloridate of formula (B) is hydrolysed to yield the phosphate of formula (C), 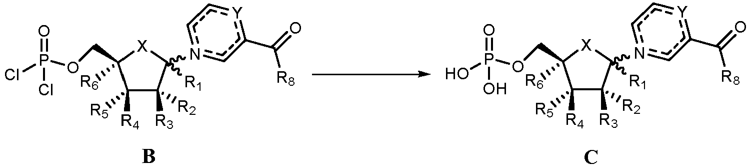 wherein X, R1, R2, R3, R4, R5, R6, R7, R8, Y, -̅ -̅ -̅ and 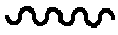 are as described herein above for compounds of formula (I).

The compound of formula (A) can be synthesized using various methods known to the person skilled in the art. According to one embodiment, the compound of formula (A) is synthesized by reacting the pentose of formula (D) with a nitrogen derivative of formula (E), wherein R, R2, R3, R4, R5, R6, R7, Y are as described above for compounds of formula (I), leading to the compound of formula (A-1) which is then selectively deprotected to give the compound of formula (A), 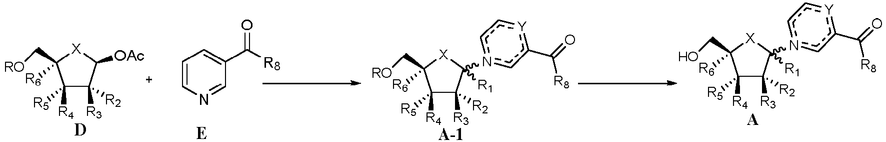 wherein X, R1, R2, R3, R4, R5, R6, R8, Y, -̅ -̅ -̅ and 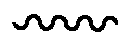 are as described herein above for compounds of formula (I).

R can be an appropriate protective group known to the skilled person in the art. In one embodiment, the protecting group is selected from triarylmethyls and/or silyls. Non-limiting examples of triarylmethyl include trityl, monomethoxytrityl, 4,4'-dimethoxytrityl and 4,4',4"-trimethoxytrityl. Non-limiting examples of silyl groups include trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

Any hydroxyl group can be attached to the pentose is protected by an appropriate protective group known to the person skilled in the art.

The choice and exchange of protective groups is the responsibility of the person skilled in the art. Protective groups can also be removed by methods well known to the skilled person, for example, with an acid (e.g. mineral or organic acid), base or fluoride source. According to a preferred embodiment, the nitrogen derivative of formula (E) is coupled to the pentose of formula (D) by a reaction in the presence of a Lewis acid leading to the compound of formula (A-1). Non-limiting examples of Lewis acids include TMSOTf, BF3.OEt2, TiCl4 and FeCl3.

The method of the present invention can further comprise a step of reducing the compound of formula (A) by various methods well known to the skilled person in the art, leading to the compound of formula (A') wherein is CH2 and R1, R2, R3, R4, R5, R6, R8, Y, -̅ -̅ -̅ and 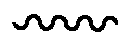 are as defined above for compounds of formula (I).

According to a specific embodiment, the present invention relates to a method for the preparation of the compounds of formula I-A to I-D.

In a first step, the nicotinamide of formula E is coupled to the ribose tetraacetate of formula D by a coupling reaction in the presence of a Lewis acid, resulting in the compound of formula A-1:

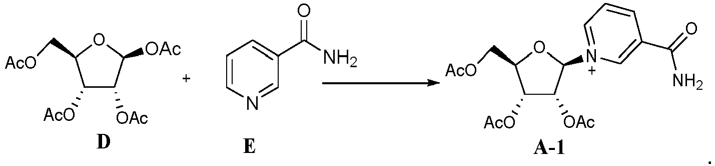

In a second step, an ammoniacal treatment of the compound of formula A-1 is carried out, leading to the compound of formula A-2:

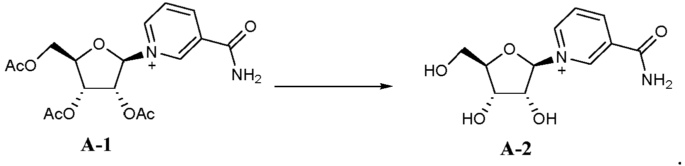

In a third step, the mono-phosphorylation of the compound of formula A-2, in the presence of phosphoryl chloride and a trialkyl phosphate, leads to the phosphorodichloridate of formula A-3:

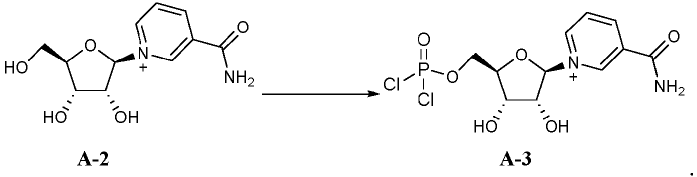

In a fourth step, the phosphorodichloridate of formula A-3 is hydrolysed to yield the compound of formula I-A:

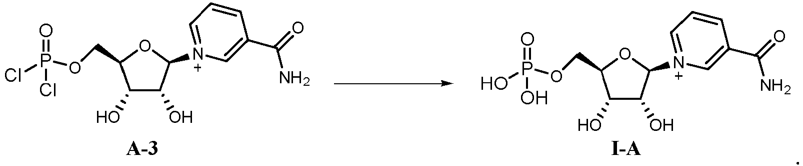

According to one embodiment, a step of reducing the compound of formula A-2 is carried out, leading to the compound of formula I-E of formula:

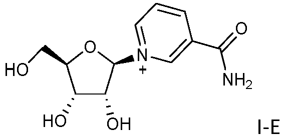

The compound of formula I-E is then monophosphorylated as described in step 4 and hydrolyzed to the compound of formula I-C.

In another aspect, the invention relates to a method for preparing compounds of formula la as described above.

In particular, compounds of formula la disclosed herein can be prepared as described below from substrates Xa-Xllla. It will be understood by one ordinary skilled in the art that these schemes are in no way limiting and that variations of detail can be made without departing from the spirit and scope of the present invention.

According to one embodiment, the invention relates to a method for preparing the compound of formula I described herein above.

The method first involves the mono-phosphorylation of a compound of formula Xa, in the presence of phosphoryl chloride in a trialkyl phosphate, to give the phophorodichloridate compound Xla, 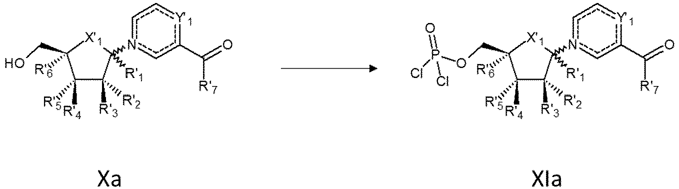 wherein X'1, R'1, R'2, R'3, R'4, R'5, R'6, R'7, Y'1, -̅ -̅ -̅ and 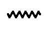 are as described herein for formula la.

In a second step the hydrolysis of the phophorodichloridate XIa obtained in the first step give the phosphate compound of formula Xlla, 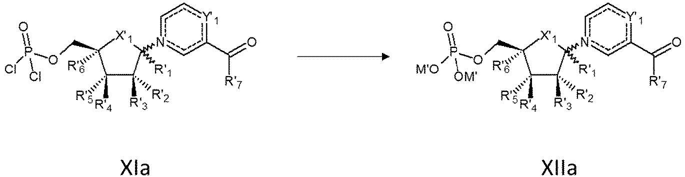 wherein X'1, R'1, R'2, R'3, R'4, R'5, R'6, R'7, Y'1, M', -̅ -̅ -̅ and 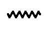 are as described herein for formula la.

The phosphate compound of formula Xlla obtained in the second step is then reacted, with a phophorodichloridate compound of formula Xllla obtained as described in the first step, 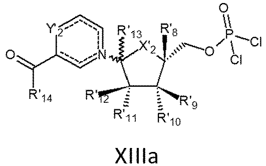 wherein X'2, R'8, R'9, R'10, R'11, R'12, R'13, R'14, Y'2, -̅ -̅ -̅ and 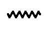 are as are as described herein for formula la, to give the compound of formula la as described herein.

According to one embodiment, the method of the invention further comprises a step of reducing the compound of formula la, using various methods known to those skilled in the art, to give the compound of formula I'a, wherein Y'1 and Y'2 are identical and represent each CH2 and wherein X'1, X'2, R'1, R'2, R'3, R'4, R'5, R'6, R'7, R'8, R'9, R'10, R'11, R'12, R'13, R'14, Y'1, Y'2, M', -̅ -̅ -̅ and 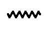 are as described herein for formula la.

According to another embodiment, the compound of formula Xa is synthesized using various methods known to those skilled in the art. According to one embodiment, the compound of formula Xa is synthesized in two steps by first reacting the pentose of formula XIVa with the nitrogenous derivatives of formula XVa, wherein R'1, R'1, R'2, R'3, R'4, R'5, R'6, R'7, Y'1 and R are as described herein for formula la, to give the compound of formula Xa-1, then selectively deprotected to give the compound of formula Xa. 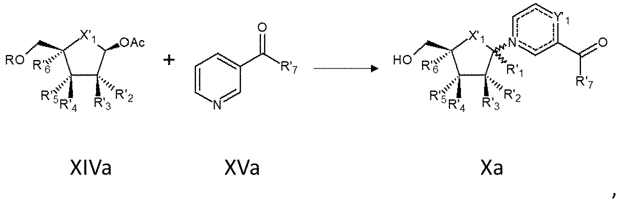 wherein X'1, R, R'1, R'2, R'3, R'4, R'5, R'6, R'7, Y'1, -̅ -̅ -̅ and 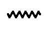 are as described herein for formula la.

According to one embodiment, R is an appropriate protecting group known to those skilled in the art. Examples of appropriate protecting group includes triarylmethyl and/or silyl groups. Non limiting examples of triarylmethyl includes trityl, monomethoxytrityl, 4,4'-dimethoxytrityl and 4,4',4"-trimethoxytrityl. Non limiting examples of silyl groups includes trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

According to one embodiment, any hydroxy group attached to the pentose ring is protected with an appropriate protecting group known to those skilled in the art.

The selection and exchange of the protecting groups is within the skill to those skilled in the art. Any protecting groups can also be removed by methods known in the art, for example, with an acid (e.g., a mineral or an organic acid), a base or a fluoride source.

According to a preferred embodiment, the nitrogenous derivatives of formula XVa is added to the pentose XIVa via a coupling reaction in the presence of a Lewis acid to give the compound of formula Xa-1. Non limiting examples of suitable Lewis acid includes TMSOTf, BF3.OEt2, TiCl4 and FeCl3.

According to a specific embodiment, the invention relates to a method for preparing the compound of formula Villa, 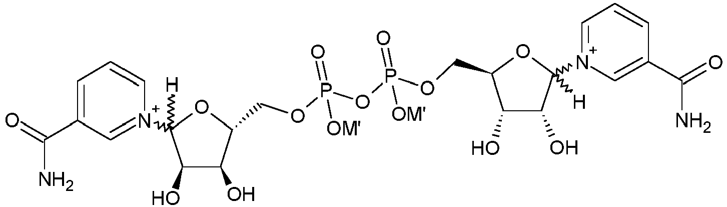 or pharmaceutically acceptable salts and/or solvates thereof.

In a first step, the nicotinamide of formula XVa, is added to the ribose tetraacetate XIVa, via a coupling reaction in the presence of a Lewis acid, to give the compound of formula Xa-1:

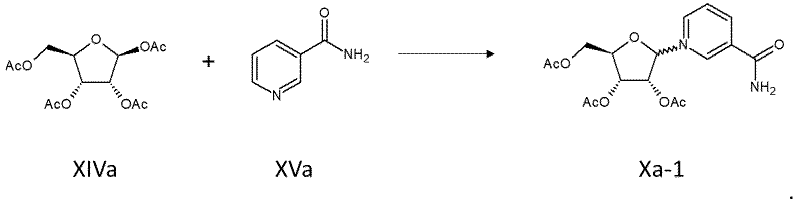

In a second step, an ammoniacal treatment of the compound of formula Xa-1 give the compound of formula Xa:

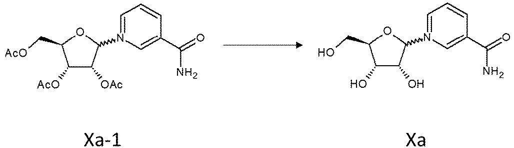

In a third step, the mono-phosphorylation of a compound of formula Xa, in the presence of phosphoryl chloride in a trialkyl phosphate, give the phophorodichloridate compound Xla:

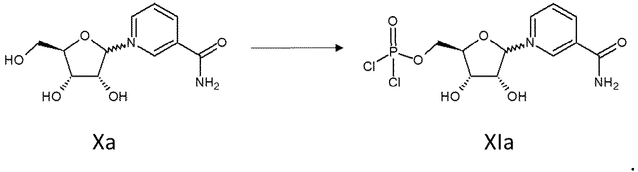

In a fourth step, the phophorodichloridate compound XIa obtained in the third step is partially hydrolyzed to give the phosphate compound of formula Xlla:

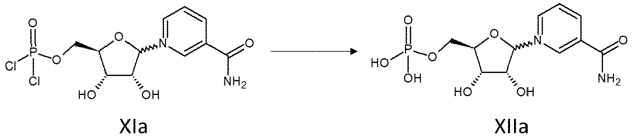

In a fifth step, the phosphate compound of formula Xlla obtained in the fourth step is then reacted, with the phophorodichloridate compound of formula Xla obtained as described in the third step, to give the compound of formula Villa.

According to another specific embodiment, the invention relates to a method for preparing the compound of formula IXa, 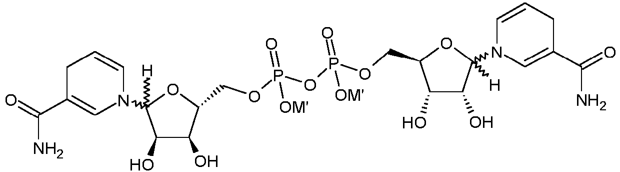 or pharmaceutically acceptable salts and/or solvates thereof.

According to one embodiment, the compound of formula IXa is obtained from the compound of formula Villa, previously synthesized as described above.

In this embodiment, the compound of formula IXa is obtained by reducing the compound of formula Villa, using a suitable reducing agent known to those skilled in the art, to give the compound of formula IXa.

### EXAMPLES

In the following description, the examples are provided to illustrate the present invention**.**

### Example 1- Administration to a 55 years old male patient having MSA.

A 55-year-old Male presenting with parkinsonism was given NMN (beta) for 6 months. The patient exhibited low response to L-dopa treatment and orthostatic hypotension before treatment. He indicated difficulties to get out of bed in the morning and feeling low and depressed during the day. Administration of 125 mg of NMN (beta) as an oral tablet occurred once per day in the morning. The patient has taken the UMSARS questionary before the beginning of the study (D0) and after 6 months of treatment. The results are shown below.

**Table 3 - UMSARS questionary - D0:**

| How many times did you experience one of the following situations during the last month? | | Never | Rarely | Sometimes | Often | Always or totally unable |
|---|---|---|---|---|---|---|
| 1 | Did you have any difficulties in the practice of your hobbies? | | | x | | |
| 2 | Have you had difficulty taking care of your home, for example: do-it-yourself, cleaning, cooking? | | | x | | |
| 3 | Did you have difficulty carrying shopping bags? | x | | | | |
| 4 | Did you have problems walking 1 km? | x | | | | |
| 5 | Did you have problems walking 100 m? | x | | | | |
| 6 | Did you have problems getting around your home as easily as you would have liked? | x | | | | |
| 7 | Have you had difficulty getting around in public places? | x | | | | |
| 8 | Did you need someone to accompany you on your outings? | x | | | | |
| 9 | Were you scared or worried about falling in public? | x | | | | |
| 10 | Have you been confined to your home more than you would have liked? | x | | | | |
| 11 | Did you have difficulty washing yourself? | x | | | | |
| 12 | Did you have difficulty getting dressed? | | x | | | |
| 13 | Have you had problems buttoning your clothes or tying your shoes? | | | x | | |
| 14 | Did you have problems writing legibly? | | | x | | |
| 15 | Did you have difficulty cutting the food? | | | x | | |
| 16 | Have you had difficulty holding a glass without spilling it? | | x | | | |
| 17 | Did you feel depressed? | | | | x | |
| 18 | Did you feel isolated and alone? | | | x | | |
| 19 | Did you feel on the verge of tears or did you cry? | | | x | | |
| 20 | Did you feel anger or bitterness? | | x | | | |
| 21 | Did you feel anxious? | | | x | | |
| 22 | Did you feel worried about your future? | | | | x | |
| 23 | Have you felt the need to hide your Parkinson's disease from others? | | x | | | |
| 24 | Have you avoided situations where you had to eat or drink in public? | x | | | | |
| 25 | Have you felt embarrassed in public because of your Parkinson's disease? | | x | | | |
| 26 | Did you feel worried about how others would react to you? | | | x | | |
| 27 | Have you had problems in your relationships with your loved ones? | | x | | | |
| 28 | Did you lack the support you needed from your spouse or partner? | | | x | | |
| 29 | Did you lack the support you needed from family or close friends? | | | x | | |
| 30 | Did you fall asleep during the day unexpectedly? | x | | | | |
| 31 | Have you had problems concentrating, for example while reading or watching television? | | x | | | |
| 32 | Did you feel that your memory was bad? | | | x | | |
| 33 | Did you have bad dreams or hallucinations? | | | x | | |
| 34 | Did you have difficulty speaking? | | x | | | |
| 35 | Have you felt unable to communicate normally with others? | | x | | | |
| 36 | Did you feel ignored by others? | | x | | | |
| 37 | Have you had painful muscle cramps or spasms? | | x | | | |
| 38 | Have you had pain or discomfort in your joints or body? | x | | | | |
| 39 | Have you had the unpleasant sensation of hot or cold? | x | | | | |

**Table 4 - UMSARS questionary - after 6 months of treatment:**

| How many times did you experience one of the following situations during the last month? | | Never | Rarely | Sometimes | Often | Always or totally unable |
|---|---|---|---|---|---|---|
| 1 | Did you have any difficulties in the practice of your hobbies? | | x | | | |
| 2 | Have you had difficulty taking care of your home, for example: do-it-yourself, cleaning, cooking? | | | x | | |
| 3 | Did you have difficulty carrying shopping bags? | x | | | | |
| 4 | Did you have problems walking 1 km? | x | | | | |
| 5 | Did you have problems walking 100 m? | x | | | | |
| 6 | Did you have problems getting around your home as easily as you would have liked? | x | | | | |
| 7 | Have you had difficulty getting around in public places? | x | | | | |
| 8 | Did you need someone to accompany you on your outings? | x | | | | |
| 9 | Were you scared or worried about falling in public? | x | | | | |
| 10 | Have you been confined to your home more than you would have liked? | x | | | | |
| 11 | Did you have difficulty washing yourself? | x | | | | |
| 12 | Did you have difficulty getting dressed? | | x | | | |
| 13 | Have you had problems buttoning your clothes or tying your shoes? | | x | | | |
| 14 | Did you have problems writing legibly? | | x | | | |
| 15 | Did you have difficulty cutting the food? | | | x | | |
| 16 | Have you had difficulty holding a glass without spilling it? | x | | | | |
| 17 | Did you feel depressed? | | | x | | |
| 18 | Did you feel isolated and alone? | | x | | | |
| 19 | Did you feel on the verge of tears or did you cry? | | | x | | |
| 20 | Did you feel anger or bitterness? | | x | | | |
| 21 | Did you feel anxious? | | | x | | |
| 22 | Did you feel worried about your future? | | | x | | |
| 23 | Have you felt the need to hide your Parkinson's disease from others? | | x | | | |
| 24 | Have you avoided situations where you had to eat or drink in public? | x | | | | |
| 25 | Have you felt embarrassed in public because of your Parkinson's disease? | | x | | | |
| 26 | Did you feel worried about how others would react to you? | | x | | | |
| 27 | Have you had problems in your relationships with your loved ones? | | x | | | |
| 28 | Did you lack the support you needed from your spouse or partner? | | x | | | |
| 29 | Did you lack the support you needed from family or close friends? | | x | | | |
| 30 | Did you fall asleep during the day unexpectedly? | x | | | | |
| 31 | Have you had problems concentrating, for example while reading or watching television? | | x | | | |
| 32 | Did you feel that your memory was bad? | | | x | | |
| 33 | Did you have bad dreams or hallucinations? | | | x | | |
| 34 | Did you have difficulty speaking? | x | | | | |
| 35 | Have you felt unable to communicate normally with others? | x | | | | |
| 36 | Did you feel ignored by others? | x | | | | |
| 37 | Have you had painful muscle cramps or spasms? | | x | | | |
| 38 | Have you had pain or discomfort in your joints or body? | x | | | | |
| 39 | Have you had the unpleasant sensation of hot or cold? | x | | | | |

Therefore, the administration of beta-NMN according to the invention improved items 1, 13, 14, 16-18, 22, 26, 28, 29, 34-36 of the UMSARS questionary. Notably, it appears that the daily administration of beta-NMN (compound IA) did not improve most of the clinical signs related to motricity apart from items 1, 13, 14 and 16. However, the daily administration improved the symptoms related to the depression observed in patients having

Therefore, the administration of the compounds according to the invention can improve the quality of life of patients having parkinsonism, especially MSA.

Example 2 - Synthesis of compound of formula I wherein Synthesis of compounds of formula I wherein R7 is 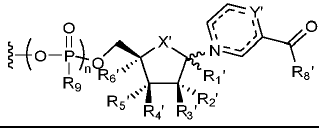 and n is 1:

As disclosed herein, a compound of formula I wherein R7 is 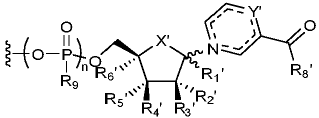 and n is 1, the synthesis can involve in a first step the mono-phosphorylation of alpha-nicotinamide riboside, in the presence of phosphoryl chloride and a trialkyl phosphate, to yield the phosphorodichloridate:

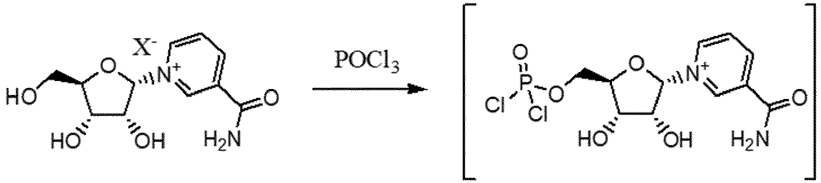

Compound I-B (alpha NMN) is then added as follows:

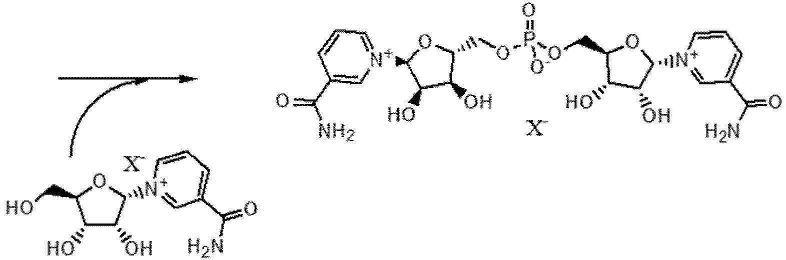

Alternatively, the compound according to the invention can be prepared by the activation of compound I-B by the addition of carbonyldiimidazole (CDI): 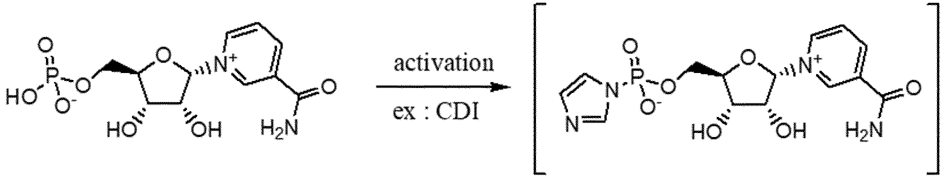 to which alpha -nicotinamide riboside is added as follows:

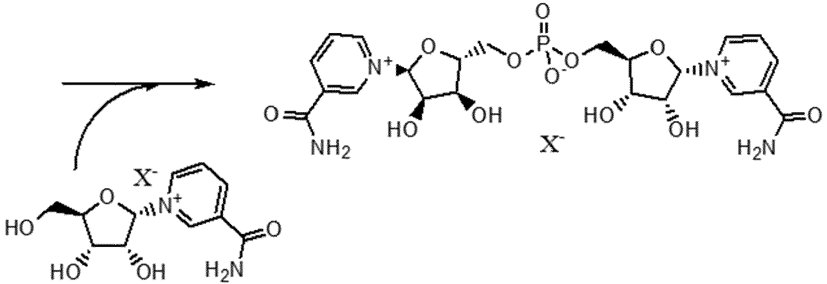

Example 3 - Synthesis of compound of formula I wherein Synthesis of compounds of formula I wherein R7 is 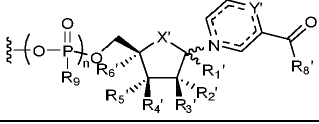 and n is 3:

A compound of formula I comprising three phosphate groups can be prepared as follows. Compound I-B can be activated by the addition of carbonyldiimidazole (CDI):

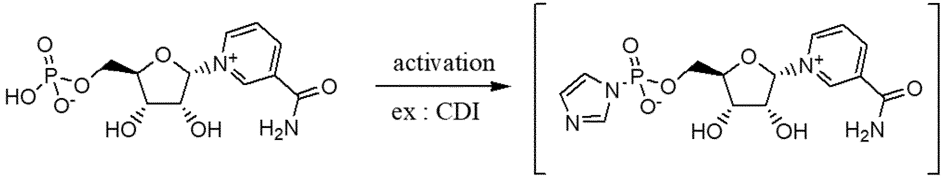

To which a tertbutyleamine -phosphate is added:

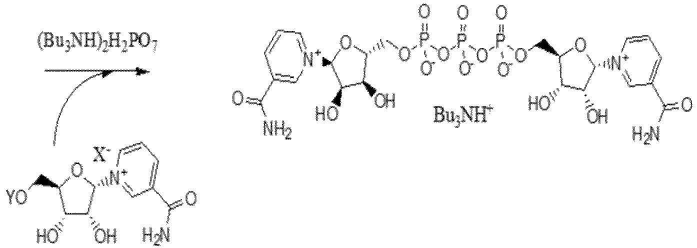

## Claims

1. A compound of formula (I) selected from :
| **Composés (anomères)** | **Structure** |
|---|---|
| I-A (beta) | 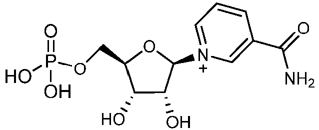 |
| I-B (alpha) | 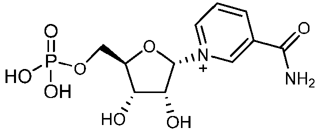 |
| I-C (beta) | 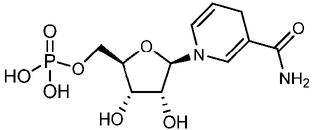 |
| I-D (alpha) | 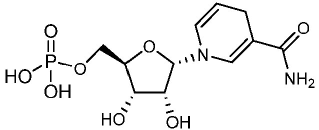 |
| I-E (beta) | 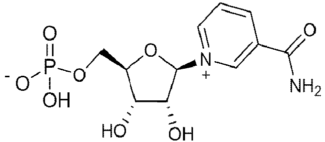 |
| I-F (alpha) | 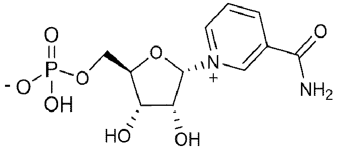 |
| I-G (beta) | 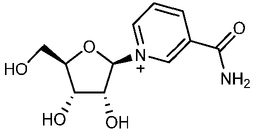 |
| I-H (alpha) | 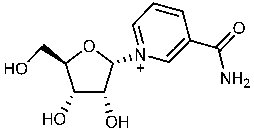 |
| I-I (beta) | 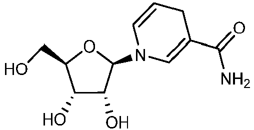 |
| I-J (alpha) | 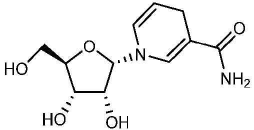 |
or pharmaceutically acceptable salts, hydrates, crystals, stereoisomers and/or solvates thereof,
or a compound of formula (Ia) selected from :
| **Compound** | **Structure** |
|---|---|
| Ia-A (beta, beta) | 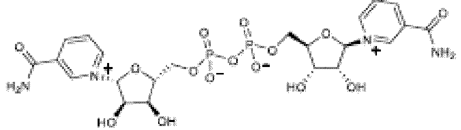 |
| Ia-B (beta, alpha) | 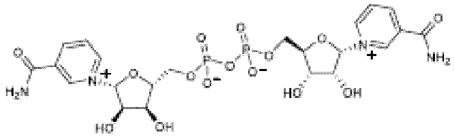 |
| Ia-C (alpha, alpha) | 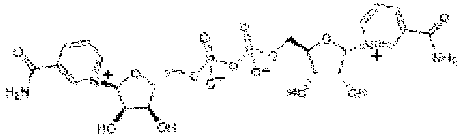 |
| Ia-D (beta, beta) | 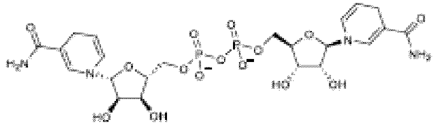 |
| Ia-E (beta, alpha) | 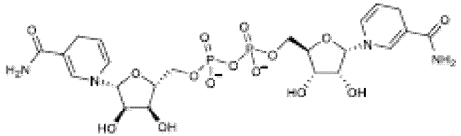 |
| Ia-F (alpha, alpha) | 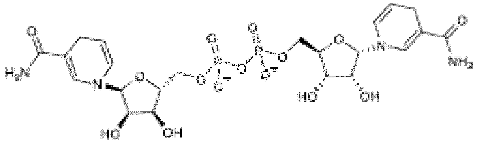 |
| Ia-G (beta, beta) | 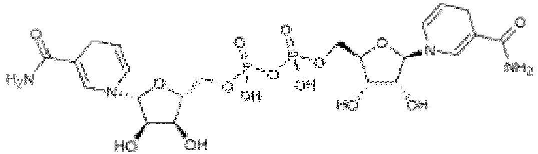 |
| Ia-H (beta, alpha) | 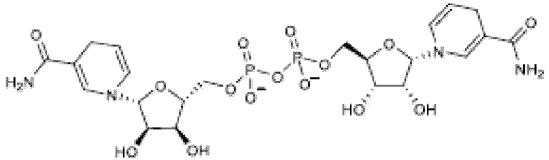 |
| Ia-I (alpha, alpha) | 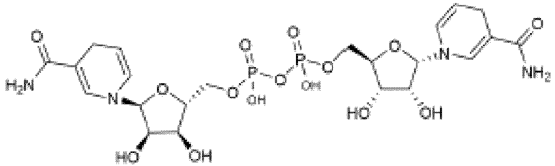 |
or pharmaceutically acceptable salts, hydrates, crystals, stereoisomers and/or solvates thereof,
and their combinations for its use in the prevention and/or treatment of depression and/or anxiety related to Parkinsonism.

2. Compound of formula (I) and/or compound of formula (Ia) for their use according to claim **1** wherein Parkinsonism is chosen amongst an alpha-synucleinopathy, a progressive supranuclear palsy, a cortico-basal degeneration, a drug induced parkinsonism and a vascular parkinsonism.

3. Compound of formula (I) and/or compound of formula (Ia) for their use according to claim **2** wherein the alpha-synucleinopathy is chosen amongst Parkinson disease (PD), multiple systemic atrophy (MSA) and dementia with Lewy bodies (DLB), preferably Parkinson disease (PD).

4. Compound of formula (I) and/or compound of formula (Ia) for their use according to claim 2 wherein the MSA is chosen amongst MSA-predominant Parkinsonism (MSA-P) or the MSA-predominant cerebellar ataxia (MSA-C).

5. Compound of formula (I) and/or compound of formula (Ia) for their use according to any of the preceding claims wherein compound of formula (I) is chosen amongst compound I-B, compound I-D or compound I-F.

6. Compound of formula (I) and/or compound of formula (Ia) for their use according to any of the preceding claims wherein compound of formula (Ia) is chosen amongst compound la-C, compound la-F, compound la-I.

7. Compound of formula (I) and/or compound of formula (Ia) for their use according to any of the preceding claims in combination with at least one further therapeutic ingredient.

8. Compound of formula (I) and/or compound of formula (Ia) for their use according to claim **7** wherein the at least one further therapeutic ingredient is chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations.

9. Compound of formula (I) and/or compound of formula (Ia) for their use according to any of the preceding claims to reduce the symptoms of depression related to parkinsonism chosen amongst anxiety, depression, sadness, anger, the sensation of lack of support from the loved ones, the feeling of being ignored and their combinations.

10. Composition comprising at least one compound of formula (I) selected from :
| **Composés (anomères)** | **Structure** |
|---|---|
| I-A (beta) | 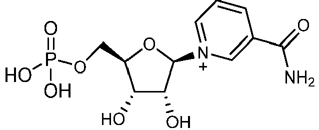 |
| I-B (alpha) | 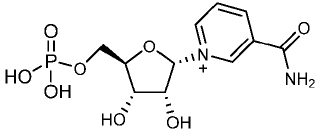 |
| I-C (beta) | 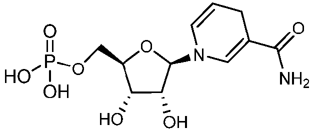 |
| I-D (alpha) | 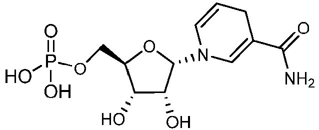 |
| I-E (beta) | 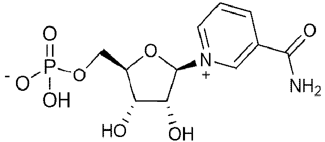 |
| I-F (alpha) | 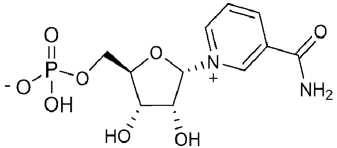 |
| I-G (beta) | 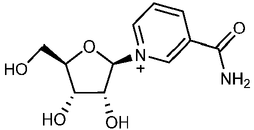 |
| I-H (alpha) | 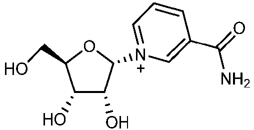 |
| I-I (beta) | 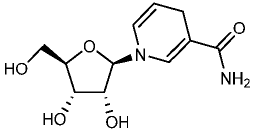 |
| I-J (alpha) | 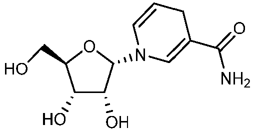 |
or pharmaceutically acceptable salts, hydrates, crystals, stereoisomers and/or solvates thereof, or a compound of formula (Ia) selected from :
| **Compound** | **Structure** |
|---|---|
| Ia-A (beta, beta) | 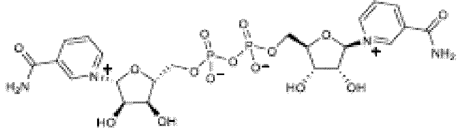 |
| Ia-B (beta, alpha) | 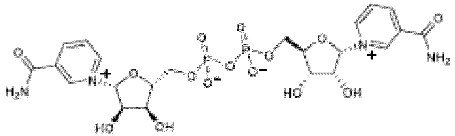 |
| Ia-C (alpha, alpha) | 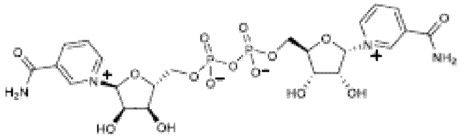 |
| Ia-D (beta, beta) | 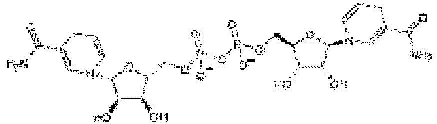 |
| Ia-E (beta, alpha) | 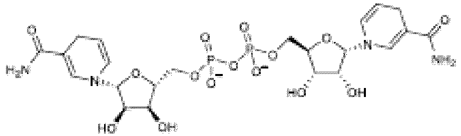 |
| Ia-F (alpha, alpha) | 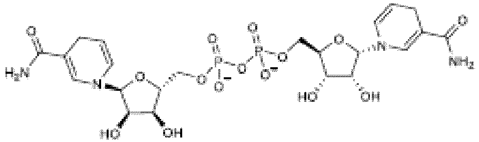 |
| Ia-G (beta, beta) | 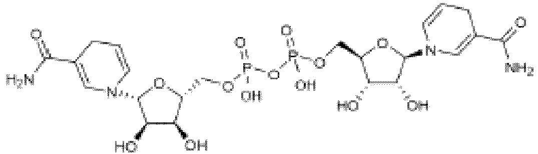 |
| Ia-H (beta, alpha) | 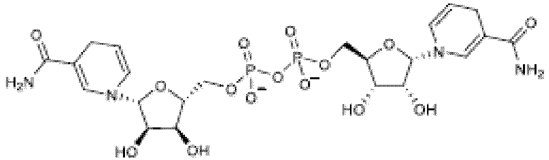 |
| Ia-I (alpha, alpha) | 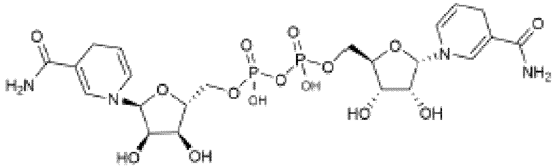 |
or pharmaceutically acceptable salts, hydrates, crystals, stereoisomers and/or solvates thereof,
and their combinations, for use in the prevention and/or treatment of depression related to Parkinsonism.

11. Composition for use according to claim **10** wherein the parkinsonism is chosen amongst an alpha-synucleinopathy, progressive supranuclear palsy, cortico-basal degeneration, drug induced parkinsonism, and vascular parkinsonism.

12. Composition for use according to claim **11** wherein parkinsonism is an alpha-synucleinopathy, preferably chosen amongst Parkinson's disease (PD), dementia with Lewy bodies (DLB) and multiple system atrophy (MSA).

13. Composition for use according to any of claims **9** to **12** further comprising at least one further therapeutic ingredient.

14. Composition for use according to claim **13** wherein the at least one further therapeutic ingredient is chosen amongst chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations.

15. Kit of parts comprising at least one compound of formula (I) and/or one compound of formula (Ia) for use according to any of claims **1** to **9** or one composition for use according to any of claims **10** to **14**, and at least one further part.

16. Kit of part for use according to claim **15** wherein the at least one further part is a further therapeutic ingredient, preferably chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations.

## Patentansprüche

1. Verbindung der Formel (I), ausgewählt aus:
| Verbindungen (Anomere) | Struktur |
|---|---|
| I-A (beta) | 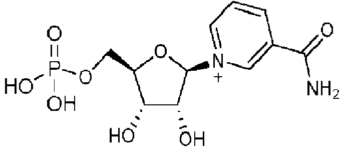 |
| I-B (alpha) | 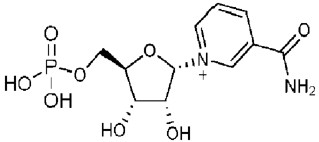 |
| I-C (beta) | 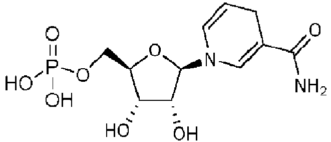 |
| I-D (alpha) | 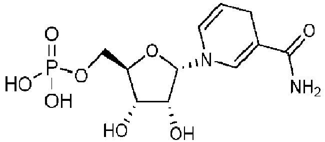 |
| I-E (beta) | 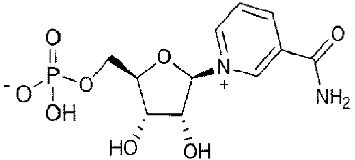 |
| I-F (alpha) | 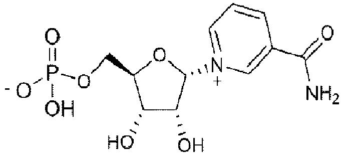 |
| I-G (beta) | 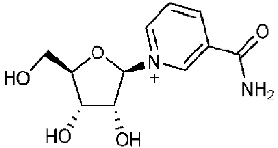 |
| I-H (alpha) | 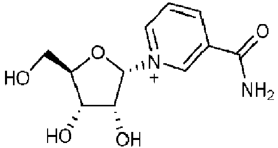 |
| I-I (beta) | 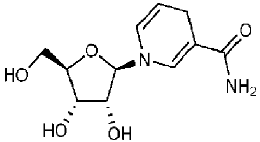 |
| I-J (alpha) | 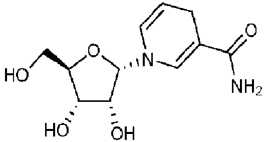 |
oder pharmazeutisch unbedenklichen Salzen, Hydraten, Kristallen, Stereoisomeren und/oder Solvaten davon,
oder eine Verbindung der Formel (la), ausgewählt aus:
| Verbindung | Struktur |
|---|---|
| Ia-A (beta, beta) | 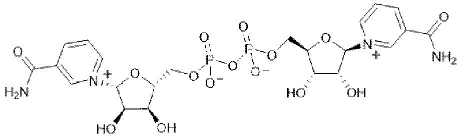 |
| Ia-B (beta, alpha) | 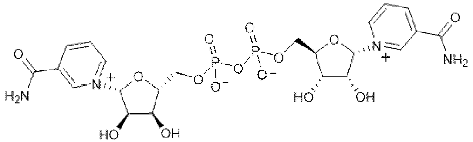 |
| Ia-C (alpha, alpha) | 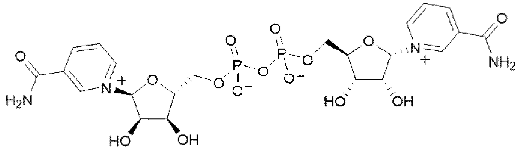 |
| Ia-D (beta, beta) | 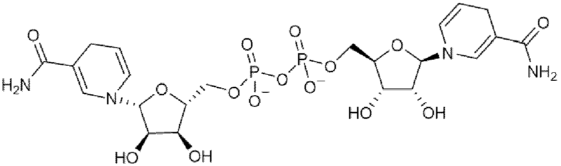 |
| Ia-E (beta, alpha) | 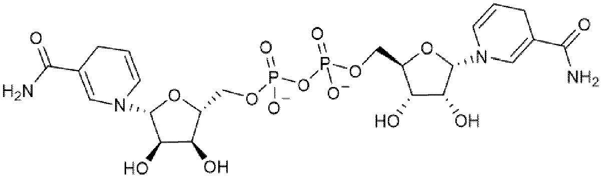 |
| Ia-F (alpha, alpha) | 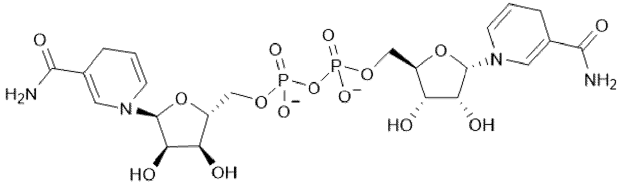 |
| Ia-G (beta, beta) | 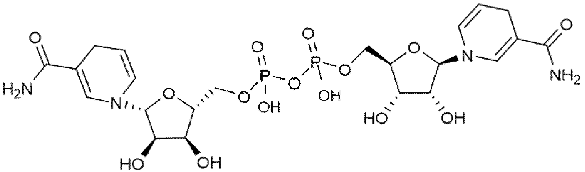 |
| Ia-H (beta, alpha) | 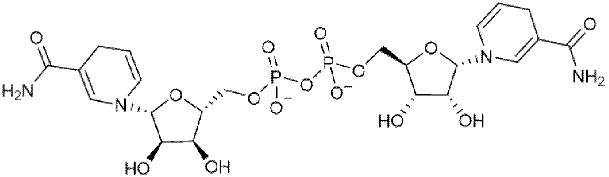 |
| Ia-I (alpha, alpha) | 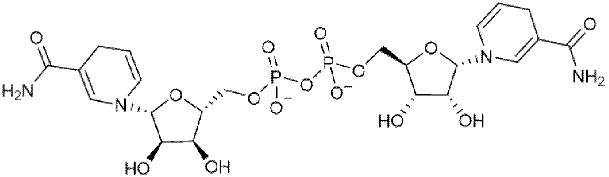 |
oder pharmazeutisch unbedenklichen Salzen, Hydraten, Kristallen, Stereoisomeren und/oder Solvaten davon,
und Kombinationen davon zur Verwendung bei der Prävention und/oder Behandlung von Depression und/oder Angst in Zusammenhang mit Parkinsonismus.

2. Verbindung der Formel (I) und/oder Verbindung der Formel (la) zur Verwendung nach Anspruch 1, wobei der Parkinsonismus aus einer alpha-Synukleinopathie, einer progressiven supranukleären Parese, einer kortikobasalen Degeneration, einem arzneimittelinduzierten Parkinsonismus und einem vaskulären Parkinsonismus ausgewählt ist.

3. Verbindung der Formel (I) und/oder Verbindung der Formel (la) zur Verwendung nach Anspruch 2, wobei die alpha-Synukleinopathie aus Morbus Parkinson (PD), Multisystematrophie (MSA) und Demenz mit Lewy-Körperchen (DLB), vorzugsweise Morbus Parkinson (PD), ausgewählt ist.

4. Verbindung der Formel (I) und/oder Verbindung der Formel (la) zur Verwendung nach Anspruch 2, wobei die MSA aus MSA mit Subtyp Parkinsonismus (MSA-P) oder MSA mit Subtyp zerebelläre Ataxie (MSA-C) ausgewählt ist.

5. Verbindung der Formel (I) und/oder Verbindung der Formel (la) zur Verwendung nach einem der vorstehenden Ansprüche, wobei die Verbindung der Formel (I) aus Verbindung I-B, Verbindung I-D oder Verbindung I-F ausgewählt ist.

6. Verbindung der Formel (I) und/oder Verbindung der Formel (la) zur Verwendung nach einem der vorstehenden Ansprüche, wobei die Verbindung der Formel (la) aus Verbindung la-C, Verbindung la-F und Verbindung Ia-I ausgewählt ist.

7. Verbindung der Formel (I) und/oder Verbindung der Formel (la) zur Verwendung nach einem der vorstehenden Ansprüche in Kombination mit zumindest einem weiteren therapeutischen Bestandteil

8. Verbindung der Formel (I) und/oder Verbindung der Formel (la) zur Verwendung nach Anspruch 7, wobei der zumindest eine weitere therapeutische Bestandteil aus Levodopa, Carbidopa, Droxidopa, Oxybutinchlorid, Tolterodin, Tamsulosin, Mirabegron, Tadalafil, Sildenafil, Midodrin, Fludrokortison, Desmopressin, einem Laxativum, einem anticholinergen Mittel, einem Hemmer von Acetylcholinesterase (ACE), einem Benzodiazepin, einem dopaminergen Mittel, Zolpidem, Modafinil, Armodafinil und Kombinationen davon ausgewählt ist.

9. Verbindung der Formel (I) und/oder Verbindung der Formel (la) zur Verwendung nach einem der vorstehenden Ansprüche, um die Symptome von Depression in Zusammenhang mit Parkinsonismus zu lindern, die aus Angst, Depression, Traurigkeit, Wut, Gefühl von mangelnder Unterstützung durch die Angehörigen, Gefühl des Ignoriertwerdens und Kombinationen davon ausgewählt sind.

10. Zusammensetzung, die zumindest eine Verbindung der Formel (I), ausgewählt aus:
| Verbindungen (Anomere) | Struktur |
|---|---|
| I-A (beta) | 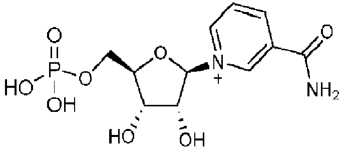 |
| I-B (alpha) | 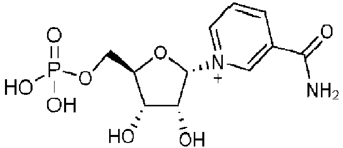 |
| I-C (beta) | 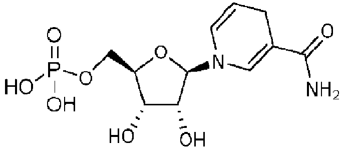 |
| I-D (alpha) | 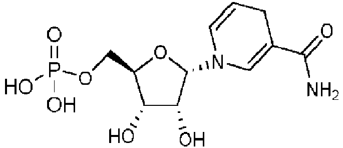 |
| I-E (beta) | 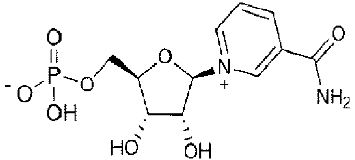 |
| I-F (alpha) | 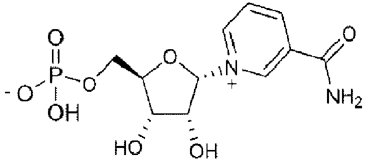 |
| I-G (beta) | 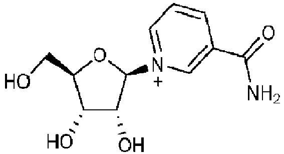 |
| I-H (alpha) | 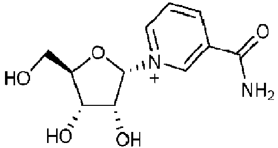 |
| I-I (beta) | 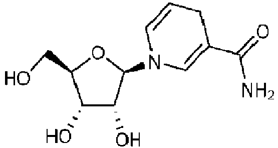 |
| I-J (alpha) | 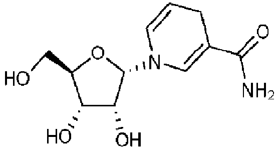 |
oder pharmazeutisch unbedenklichen Salzen, Hydraten, Kristallen, Stereoisomeren und/oder Solvaten davon, oder eine Verbindung der Formel (la) umfasst, ausgewählt aus:
| Verbindung | Struktur |
|---|---|
| Ia-A (beta, beta) | 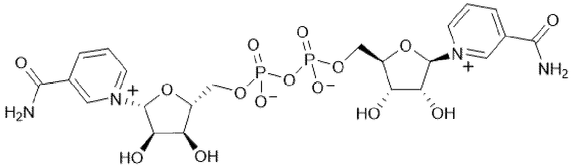 |
| Ia-B (beta, alpha) | 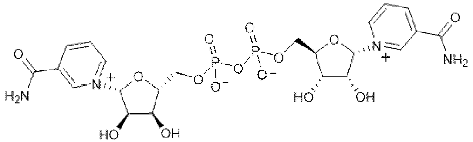 |
| Ia-C (alpha, alpha) | 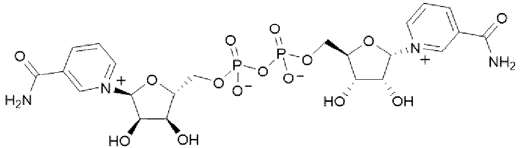 |
| Ia-D (beta, beta) | 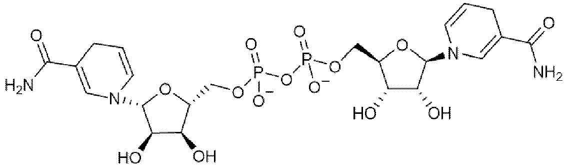 |
| Ia-E (beta, alpha) | 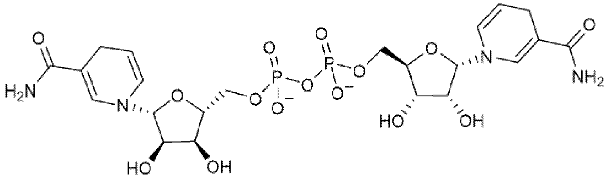 |
| Ia-F (alpha, alpha) | 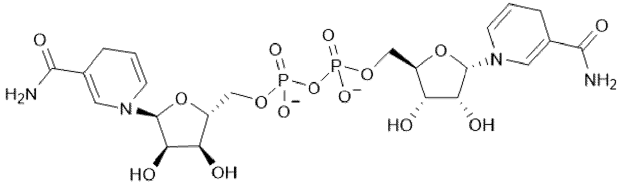 |
| Ia-G (beta, beta) | 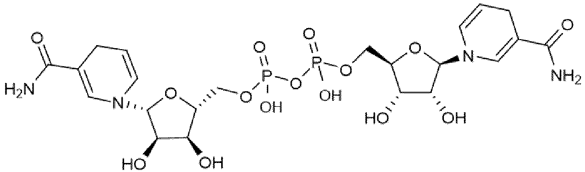 |
| Ia-H (beta, alpha) | 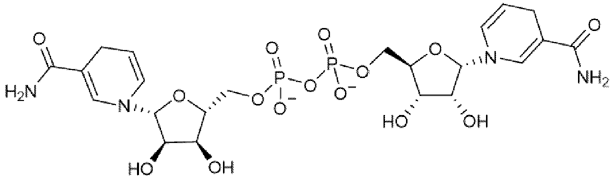 |
| Ia-I (alpha, alpha) | 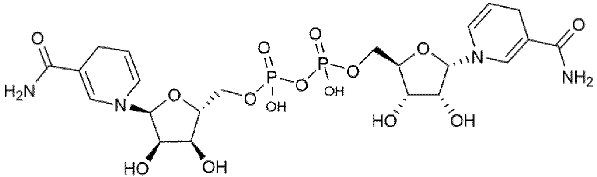 |
oder pharmazeutisch unbedenklichen Salzen, Hydraten, Kristallen, Stereoisomeren und/oder Solvaten davon,
und Kombinationen davon umfasst, zur Verwendung bei der Prävention und/oder Behandlung von Depression und/oder Angst in Zusammenhang mit Parkinsonismus.

11. Zusammensetzung zur Verwendung nach Anspruch 10, wobei der Parkinsonismus aus einer alpha-Synukleinopathie, einer progressiven supranukleären Parese, einer kortikobasalen Degeneration, einem arzneimittelinduzierten Parkinsonismus und einem vaskulären Parkinsonismus ausgewählt ist.

12. Zusammensetzung zur Verwendung nach Anspruch 11, wobei der Parkinsonismus eine alpha-Synukleinopathie ist, die vorzugsweise aus Morbus Parkinson (PD), Demenz mit Lewy-Körperchen (DLB) und Multisystematrophie (MSA) ausgewählt ist.

13. Zusammensetzung zur Verwendung nach einem der Ansprüche 9 bis 12, die ferner zumindest einen weiteren therapeutischen Bestandteil umfasst.

14. Zusammensetzung zur Verwendung nach Anspruch 13, wobei der zumindest eine weitere therapeutische Bestandteil aus Levodopa, Carbidopa, Droxidopa, Oxybutinchlorid, Tolterodin, Tamsulosin, Mirabegron, Tadalafil, Sildenafil, Midodrin, Fludrokortison, Desmopressin, einem Laxativum, einem anticholinergen Mittel, einem Hemmer von Acetylcholinesterase (ACE), einem Benzodiazepin, einem dopaminergen Mittel, Zolpidem, Modafinil, Armodafinil und Kombinationen davon ausgewählt ist.

15. Teilekit, der zumindest eine Verbindung der Formel (I) und/oder eine Verbindung der Formel (la) zur Verwendung nach einem der Ansprüche 1 bis 9 oder eine Zusammensetzung zur Verwendung nach einem der Ansprüche 10 bis 14 und zumindest einen weiteren Teil umfasst.

16. Teilekit zur Verwendung nach Anspruch 15, wobei der zumindest eine weitere Teil ein weiterer therapeutischer Bestandteil ist, der vorzugsweise aus Levodopa, Carbidopa, Droxidopa, Oxybutinchlorid, Tolterodin, Tamsulosin, Mirabegron, Tadalafil, Sildenafil, Midodrin, Fludrokortison, Desmopressin, einem Laxativum, einem anticholinergen Mittel, einem Hemmer von Acetylcholinesterase (ACE), einem Benzodiazepin, einem dopaminergen Mittel, Zolpidem, Modafinil, Armodafinil und Kombinationen davon ausgewählt ist.

## Revendications

1. Composé de formule (I) choisi parmi :
| Composés (anomères) | Structure |
|---|---|
| I-A (bêta) | 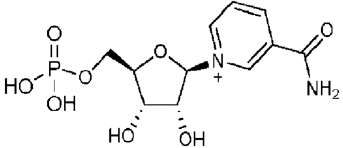 |
| I-B (alpha) | 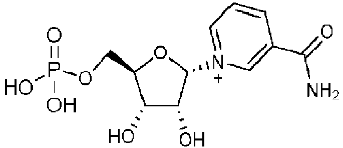 |
| I-C (bêta) | 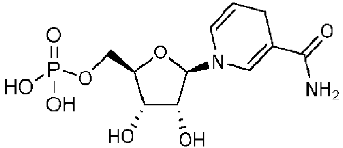 |
| I-D (alpha) | 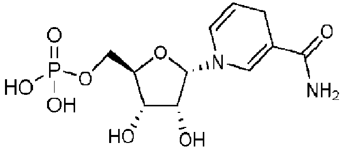 |
| I-E (bêta) | 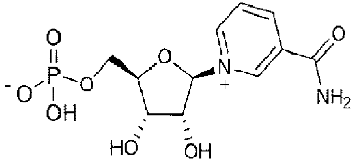 |
| I-F (alpha) | 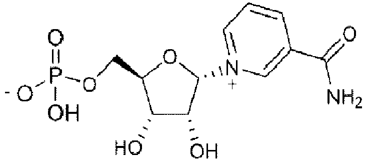 |
| I-G (bêta) | 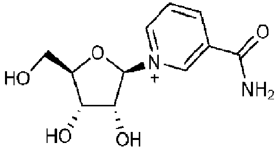 |
| I-H (alpha) | 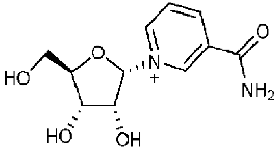 |
| I-I (bêta) | 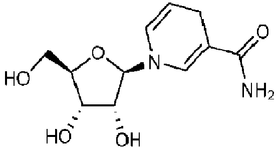 |
| I-J (alpha) | 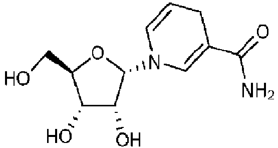 |
ou ses sels, hydrates, cristaux, stéréoisomères et/ou solvates pharmaceutiquement acceptables,
ou un composé de formule (la) choisi parmi :
| Composé | Structure |
|---|---|
| la-A (bêta, bêta) | 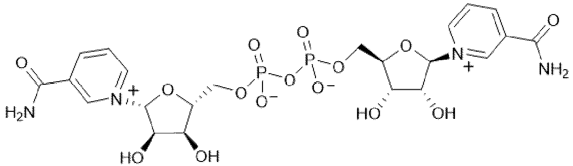 |
| la-B (bêta, alpha) | 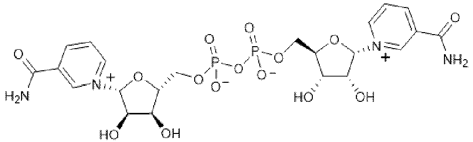 |
| la-C (alpha, alpha) | 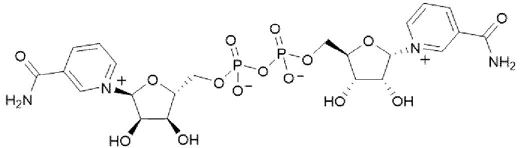 |
| la-D (bêta, bêta) | 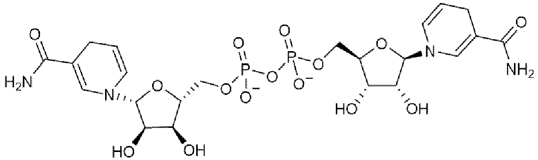 |
| la-E (bêta, alpha) | 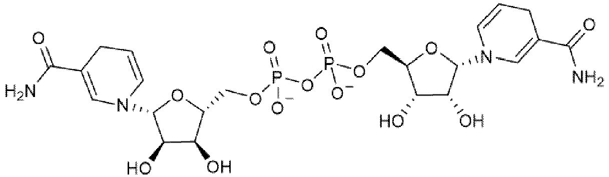 |
| la-F (alpha, alpha) | 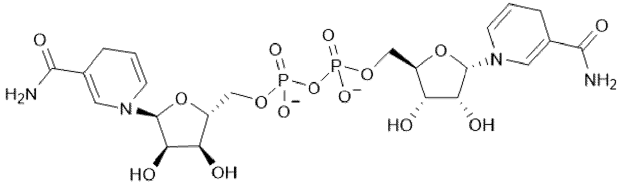 |
| la-G (bêta, bêta) | 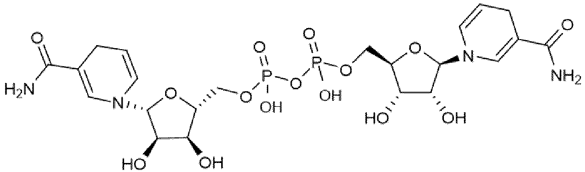 |
| la-H (bêta, alpha) | 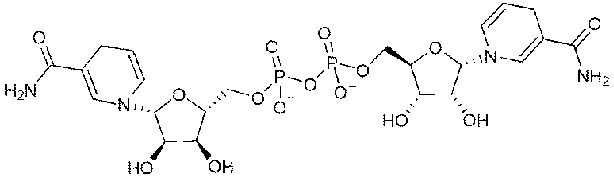 |
| la-I (alpha, alpha) | 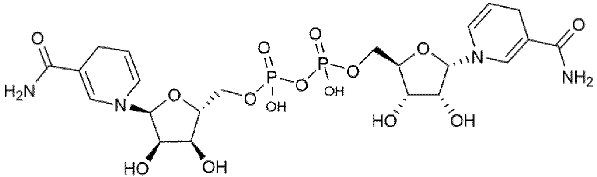 |
ou ses sels, hydrates, cristaux, stéréoisomères et/ou solvates pharmaceutiquement acceptables,
et leurs combinaisons, pour leur utilisation dans la prévention et/ou le traitement d'une dépression et/ou d'une anxiété associées à un parkinsonisme.

2. Le composé de formule (I) et/ou composé de formule (la) pour leur utilisation selon la revendication 1, dans lesquels le parkinsonisme est choisi parmi une alpha-synucléinopathie, une ophtalmoplégie supranucléaire progressive, une dégénérescence cortico-basale, un parkinsonisme médicamenteux et un parkinsonisme vasculaire.

3. Le composé de formule (I) et/ou composé de formule (la) pour leur utilisation selon la revendication 2, dans lesquels l'alpha-synucléinopathie est choisie parmi la maladie de Parkinson (MP), l'atrophie multisystématisée (AMS) et la démence à corps de Lewy (DCL), de préférence la maladie de Parkinson (MP).

4. Le composé de formule (I) et/ou composé de formule (la) pour leur utilisation selon la revendication 2, dans lesquels l'AMS est choisie parmi l'AMS de type parkinsonien (AMS-P) et l' AMS de type cérébelleux (AMS-C).

5. Le composé de formule (I) et/ou composé de formule (la) pour leur utilisation selon l'une quelconque des revendications précédentes, dans lesquels le composé de formule (I) est choisi parmi le composé I-B, le composé I-D et le composé I-F.

6. Le composé de formule (I) et/ou composé de formule (la) pour leur utilisation selon l'une quelconque des revendications précédentes, dans lesquels le composé de formule (Ia) est choisi parmi le composé la-C, le composé la-F, le composé Ia-I.

7. Le composé de formule (I) et/ou composé de formule (la) pour leur utilisation selon l'une quelconque des revendications précédentes, en combinaison avec au moins un autre ingrédient thérapeutique.

8. Le composé de formule (I) et/ou composé de formule (la) pour leur utilisation selon la revendication 7, dans lequel l'au moins un autre ingrédient thérapeutique est choisi parmi la lévodopa, la carbidopa, la droxidopa, le chlorure d'oxybutine, la toltérodine, la tamsulosine, le mirabégron, le tadalafil, le sildénafil, la midodrine, la fludrocortisone, la desmopressine, un laxatif, un agent anticholinergique, un inhibiteur d'acétylcholinestérase (ACE), une benzodiazépine, un agent dopaminergique, le zolpidem, le modafinil, l'armodafinil et leurs combinaisons.

9. Le composé de formule (I) et/ou composé de formule (la) pour leur utilisation selon l'une quelconque des revendications précédentes pour réduire les symptômes de dépression associée à un parkinsonisme choisis parmi une anxiété, une dépression, une tristesse, une colère, la sensation de manque de soutien des êtres chers, la sensation d'être ignoré, et leurs combinaisons.

10. Composition comprenant au moins un composé de formule (I) choisi parmi :
| Composés (anomères) | Structure |
|---|---|
| l-A (bêta) | 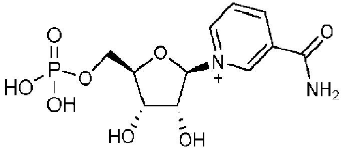 |
| l-B (alpha) | 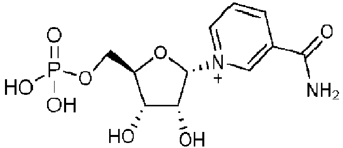 |
| l-C (bêta) | 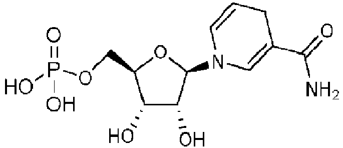 |
| l-D (alpha) | 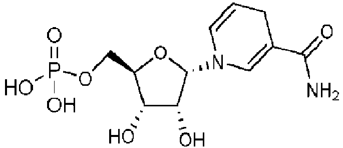 |
| l-E (bêta) | 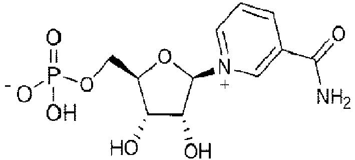 |
| l-F (alpha) | 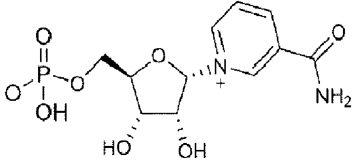 |
| l-G (bêta) | 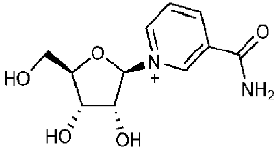 |
| l-H (alpha) | 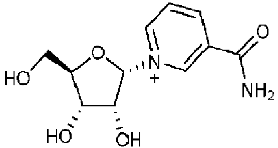 |
| l-I (bêta) | 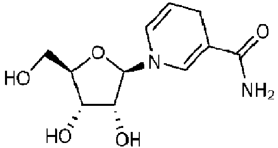 |
| l-J (alpha) | 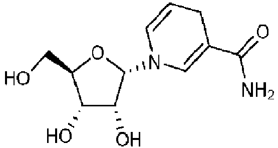 |
ou ses sels, hydrates, cristaux, stéréoisomères et/ou solvates pharmaceutiquement acceptables,
ou un composé de formule (la) choisi parmi :
| Composé | Structure |
|---|---|
| la-A (bêta, bêta) | 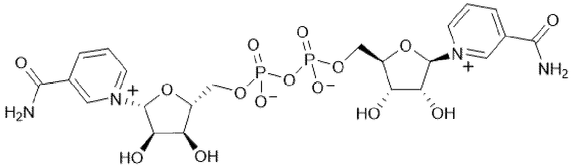 |
| la-B (bêta, alpha) | 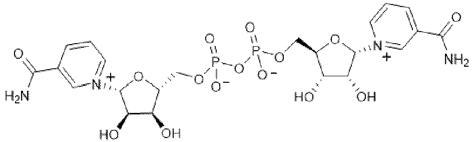 |
| la-C (alpha, alpha) | 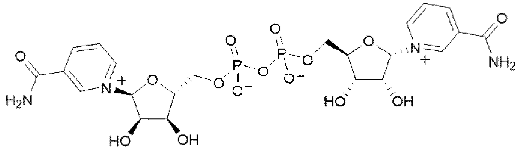 |
| la-D (bêta, bêta) | 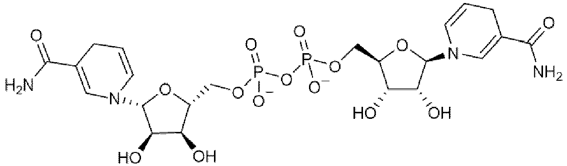 |
| la-E (bêta, alpha) | 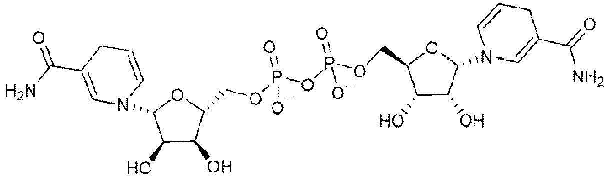 |
| la-F (alpha, alpha) | 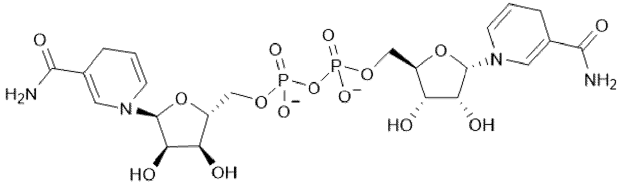 |
| la-G (bêta, bêta) | 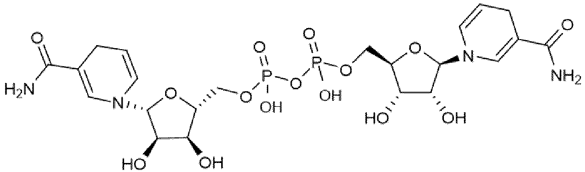 |
| la-H (bêta, alpha) | 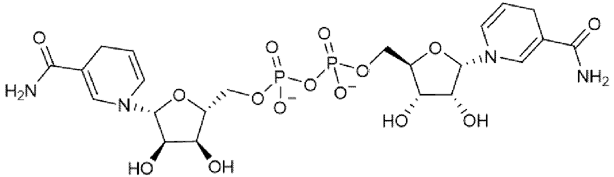 |
| la-I (alpha, alpha) | 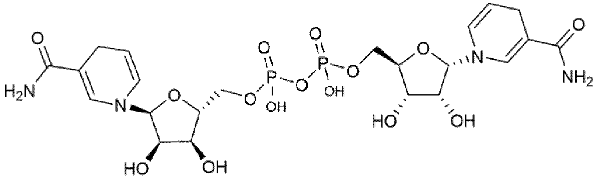 |
ou ses sels, hydrates, cristaux, stéréoisomères et/ou solvates pharmaceutiquement acceptables,
et leurs combinaisons, pour une utilisation dans la prévention et/ou le traitement d'une dépression associée à un parkinsonisme.

11. La composition pour une utilisation selon la revendication 10, dans laquelle le parkinsonisme est choisi parmi une alpha synucléinopathie, une ophtalmoplégie supranucléaire progressive, une dégénérescence cortico-basale, un parkinsonisme médicamenteux et un parkinsonisme vasculaire.

12. La composition pour une utilisation selon la revendication 11, dans laquelle le parkinsonisme est une alpha-synucléinopathie de préférence choisie parmi la maladie de Parkinson (MP), la démence à corps de Lewy (DCL) et l'atrophie multisystématisée (AMS).

13. Composition pour une utilisation selon l'une quelconque des revendications 9 à 12, comprenant en outre au moins un autre ingrédient thérapeutique.

14. Composition pour une utilisation selon la revendication 13, dans laquelle l'au moins un autre ingrédient thérapeutique est choisi parmi la lévodopa, la carbidopa, la droxidopa, le chlorure d'oxybutine, la toltérodine, la tamsulosine, le mirabégron, le tadalafil, le sildénafil, la midodrine, la fludrocortisone, la desmopressine, un laxatif, un agent anticholinergique, un inhibiteur d'acétylcholinestérase (ACE), une benzodiazépine, un agent dopaminergique, le zolpidem, le modafinil, l'armodafinil et leurs combinaisons.

15. Kit d'éléments comprenant au moins un composé de formule (I) et/ou un composé de formule (la) pour une utilisation selon l'une quelconque des revendications 1 à 9 ou une composition pour une utilisation selon l'une quelconque des revendications 10 à 14 et au moins un autre élément.

16. Kit d'éléments pour une utilisation selon la revendication 15, dans laquelle l'au moins un autre élément est un autre ingrédient thérapeutique, de préférence choisi parmi la lévodopa, la carbidopa, la droxidopa, le chlorure d'oxybutine, la toltérodine, la tamsulosine, le mirabégron, le tadalafil, le sildénafil, la midodrine, la fludrocortisone, la desmopressine, un laxatif, un agent anticholinergique, un inhibiteur d'acétylcholinestérase (ACE), une benzodiazépine, un agent dopaminergique, le zolpidem, le modafinil, l'armodafinil et leurs combinaisons.
